# FASCICULE DE BREVET EUROPEEN

(11) **EP 3 339 314 B1**
(45) Date de publication et mention de la délivrance du brevet: **05.05.2021**
(21) Numéro de dépôt: 18153938.8
(22) Date de dépôt: 01.09.2015
(51) Int. Cl.: C07H 19/10, C07H 19/20, C12P 19/34

(54) **NUCLEOTIDES MODIFIES POUR LA SYNTHESE D'ACIDES NUCLEIQUES, UN KIT RENFERMANT DE TELS NUCLEOTIDES ET LEUR UTILISATION POUR LA PRODUCTION DE GENES OU SEQUENCES D'ACIDES NUCLEIQUES SYNTHETIQUES**
VERÄNDERTE NUKLEOTIDE FÜR DIE SYNTHESE VON NUKLEINSÄUREN, KIT, DAS DIESE NUKLEOTIDE UMFASST, UND IHRE VERWENDUNG ZUR HERSTELLUNG VON GENEN ODER SYNTHETISCHEN NUKLEINSÄURESEQUENZEN
MODIFIED NUCLEOTIDES FOR NUCLEIC ACID SYNTHESIS, KIT COMPRISING SUCH NUCLEOTIDES AND USE THEREOF FOR THE PRODUCTION OF SYNTHETIC NUCLEIC ACID SEQUENCES OR GENES

(30) Priorité: 02.09.2014 FR 1458194
(43) Date de publication de la demande: 27.06.2018
(62) Demande divisionnaire de: 15766907.8
(73) Titulaire: DNA Script, 75005 Paris (FR)
(72) Inventeur: YBERT, Thomas, 75012 PARIS (FR); GARIEL, Sylvain, 75003 PARIS (FR)
(74) Mandataire: Cabinet Becker et Associés

(56) Documents cités:
- GULILAT GEBEYEHU ET AL: "Novel biotinylated nucleotide analogs for labeling and colorimetric detection of DNA", NUMBER 15 (11), vol. 15, no. 11, 1 janvier 1987 (1987-01-01), pages 4513-4534, XP055181701, DOI: 10.1093/nar/15.11.4513
- ANILKUMAR R. KORE ET AL: "Synthesis and Activity of Modified Cytidine 5'-Monophosphate Probes for T4 RNA Ligase 1", NUCLEOSIDES, NUCLEOTIDES & NUCLEIC ACIDS, vol. 28, no. 4, 22 mai 2009 (2009-05-22), pages 292-302, XP055181704, ISSN: 1525-7770, DOI: 10.1080/15257770902946181
- J.L. FLICKINGER ET AL: "Differential incorporation of biotinylated nucleotides by terminal deoxynucleotidyl transferase", NUCLEIC ACIDS RESEARCH, vol. 20, no. 9, 1 janvier 1992 (1992-01-01), pages 2382-2382, XP055181706, ISSN: 0305-1048, DOI: 10.1093/nar/20.9.2382
- PETRIE C R ET AL: "A NOVEL BIOTINYLATED ADENYLATE ANALOGUE DERIVED FROM PYRAZOLOÚ3,4-D 3/4 PYRIMIDINE FOR LABELING DNA PROBES", BIOCONJUGATE CHEMISTRY, ACS, WASHINGTON, DC, US, vol. 2, no. 6, 1 novembre 1991 (1991-11-01), pages 441-446, XP000572789, ISSN: 1043-1802, DOI: 10.1021/BC00012A011
- BEABEALASHVILLI R S ET AL: "Nucleoside 5'-triphosphates modified at sugar residues as substrates for calf thymus terminal deoxynucleotidyl transferase and for AMV reverse transcriptase", BIOCHIMICA ET BIOPHYSICA ACTA . GENE STRUCTURE AND EXPRESSION, ELSEVIER, AMSTERDAM, NL, vol. 868, no. 2-3, 13 novembre 1986 (1986-11-13), pages 136-144, XP025209880, ISSN: 0167-4781, DOI: 10.1016/0167-4781(86)90016-3 [extrait le 1986-11-13]

## Description

La présente invention relève du domaine de la synthèse de copolymères fonctionnalisés d'intérêt biologique. Elle concerne plus particulièrement des nucléotides nécessaires à la synthèse d'acides nucléiques, notamment d'acides nucléiques de grande longueur, un kit renfermant de tels nucléotides et leur utilisation pour la production de gènes ou séquences d'acides nucléiques synthétiques.

### État de la technique antérieure

A ce jour il existe deux grandes catégories de synthèse *in vitro* des acides nucléiques : les synthèses chimiques ou les synthèses enzymatiques.

La méthode de synthèse chimique des acides nucléiques *in vitro* la plus courante est la méthode de polymérisation au moyen de phosphoramidites décrite par Adams et al. (1983, J. Amer. Chem. Soc. 105:661) et Froehler et al. (1983, Tetrahedron Lett. 24:3171). Dans cette méthode, chaque nucléotide à ajouter est protégé au niveau du groupe 5'-OH de façon à éviter une polymérisation incontrôlée de plusieurs nucléotides du même type. Généralement la protection du groupe 5'-OH est réalisée par un groupement trityle. Afin d'éviter une éventuelle dégradation due à l'utilisation de réactifs puissants, les bases portées par les nucléotides peuvent également être protégées. Généralement la protection utilisée fait intervenir un groupement isobutyryle (Reddy et al. 1997, Nucleosides & Nucleotides 16:1589). Après chaque incorporation de nouveaux nucléotides, le groupe 5'-OH du dernier nucléotide de la chaîne subit une réaction de déprotection en vue de le rendre disponible pour l'étape suivante de polymérisation. Les bases azotées portées par les nucléotides composant l'acide nucléique, elles, ne sont déprotégées qu'après achèvement de la polymérisation complète.

Ces méthodes de synthèses chimiques se révèlent coûteuses et dangereuses à utiliser en raison de la nature des réactifs mis en jeux. De plus elles sont inefficaces pour la synthèse de longs fragments d'acides nucléiques (fragments supérieurs à une centaine de nucléotides).

En vue de développer des méthodes de synthèse d'acides nucléiques, notamment pour la production de fragments de grande longueur avec des rendements élevés, ainsi que des méthodes compatibles avec les éléments génétiques déjà existants, tels que les plasmides d'ADN, ont été mises au point des techniques de synthèse utilisant des catalyseurs enzymatiques pour réaliser la réaction de couplage entre les nucléotides, même en l'absence de brin matrice.

Dans certaines de ces méthodes de synthèses enzymatiques, l'enzyme permettant la polymérisation est directement ajouté aux nucléotides naturels (Deng et al. 1983, Meth. Enzymol. 100 :96). A partir d'un fragment d'acide nucléique initial appelé primer, l'enzyme de polymérisation ainsi que des nucléotides d'un même type sont ajoutés. La réaction de polymérisation est alors amorcée, l'acide nucléique grandit de façon séquentielle par répétition de ces étapes de création de liaison phosphodiester, jusqu'à ce que ladite polymérisation soit arrêtée par une méthode physique ou chimique. L'utilisation de nucléotides naturels (c'est-à-dire non modifiés et non protégés) entraine une polymérisation incontrôlée aboutissant à un mélange très hétérogène de molécules d'acides nucléiques. En effet rien ne prévient l'addition de plusieurs nucléotides d'un même type après une première addition. Dans la pratique une telle méthode de synthèse se révèle inutilisable pour la synthèse de fragments d'acide nucléiques ayant une séquence désirée.

L'utilisation de nucléotides protégés permet dans une certaine mesure de résoudre ce phénomène de polymérisation incontrôlée. Les nucléotides protégés permettent l'arrêt de la synthèse en empêchant de manière totale ou partielle la création de liaisons phosphodiester ultérieures à celle désirée.

Les nucléotides sont les « monomères » utilisés pour la synthèse d'acides nucléiques. Leurs propriétés chimiques ainsi que leur capacité à réagir ou non sont garantes du bon déroulement de la synthèse désirée. De façon à pouvoir synthétiser un fragment d'acide nucléique comportant la séquence voulue, il est important de pouvoir polymériser un à un les nucléotides dans l'ordre désiré. Cette polymérisation équivaut à l'addition de nucléotides les uns après les autres dans un ordre qui doit être strictement respecté. Il faut notamment veiller à ce que plusieurs nucléotides comportant la même base azotée et introduits au même instant ne réagissent pas en chaîne, entraînant la croissance incontrôlée de la chaîne oligomérique et de ce fait l'obtention d'une séquence erronée d'acide nucléique.

Il existe des nucléotides modifiés comportant certaines modifications structurelles par rapport aux nucléotides naturels, qui leur confèrent certains avantages lors de leur utilisation pour la synthèse d'acides nucléiques. Ils sont généralement obtenus par modifications chimiques ou enzymatiques des nucléotides naturellement présents dans les cellules. Certains nucléotides modifiés sont dits protégés car ils comportent des groupements chimiques interdisant la modification d'une fonction chimique à préserver au cours d'autres réactions. Les groupements protecteurs peuvent être disposés à différents endroits de la molécule du nucléotide.

Une classe particulière de nucléotides protégés possède une fonction de terminaison de la réaction de polymérisation. Le rôle de ces nucléotides « terminateurs de chaîne» consiste à empêcher la polymérisation excessive et non désirée des nucléotides introduits dans le milieu réactionnel. Lorsqu'un nucléotide terminateur est incorporé à une molécule d'acide nucléique, il entrave la polymérisation ultérieure d'un autre nucléotide. Ainsi, un seul nucléotide peut être ajouté à chaque molécule d'acide nucléique lors de l'étape d'élongation. Même si les différents nucléotides, composant le fragment d'acide nucléique à synthétiser, sont introduits séquentiellement il est nécessaire d'utiliser des nucléotides « terminateurs » pour éviter les phénomènes de répétitions indésirables.
L'emploi de nucléotides « terminateurs » garantit la fiabilité et la reproductibilité des méthodes de synthèse d'acides nucléiques, qu'elles soient chimiques ou enzymatiques. Ils peuvent avoir une grande influence sur les performances de synthèse d'une méthode donnée.

Les nucléotides protégés employés pour la synthèse chimique d'acides nucléiques comportent en position 5'-OH une protection par liaison covalente à un groupe DMT (4,4'-diméthoxytrityle) et en position 3'-OH un groupe phosphoramidite jouant le rôle de catalyseur de la réaction de polymérisation des nucléotides entre eux. Ces nucléotides comportant les groupes DMT et phosphoramidite sont appelés nucléotides phosphoramidites protégés. La protection contre la polymérisation incontrôlée est assurée par le groupe DMT protégeant le 5'-OH. Lors de la synthèse d'acides nucléiques par voie chimique, une première phase de déprotection appelée dé-tritylation intervient afin de retirer le groupement DMT et obtenir un groupe 5'-OH disponible pour réagir avec le nucléotide à insérer. Il est particulièrement important d'avoir une réaction de déprotection la plus efficace possible afin de permettre l'ajout du nucléotide suivant dans la totalité des cas.

Les nucléotides phosphoramidites protégés sont exclusivement employés lors de la synthèse chimique d'acides nucléiques. Leur fonction de "terminateur" est en effet assurée par le groupement DMT lié au 5'-OH. La synthèse chimique s'effectuant dans le sens 3' vers 5', l'existence d'un groupement DMT protecteur du 5'-OH permet d'éviter toute polymérisation excessive jusqu'à l'étape suivante de déprotection.
Ainsi les nucléotides phosphoramidites protégés ne conviennent pas aux méthodes de synthèses enzymatiques.

Certains nucléotides « terminateurs » ont aussi été développés pour des méthodes de séquençage dites de seconde génération. Cependant, en plus d'être totalement inadaptés à la synthèse d'acide nucléique, les nucléotides terminateurs employés pour le séquençage possèdent un certain nombre de limitations rédhibitoires.
La principale limitation est leur capacité à être utilisés par les enzymes d'élongation. En effet les marqueurs fluorescents liés aux nucléotides terminateurs pour le séquençage ont une taille importante. Or les enzymes d'élongation possèdent extrêmement peu d'espace au sein de leur site actif et ont donc peu de chance de pouvoir accepter, afin de les polymériser, des nucléotides terminateurs arborant d'imposants groupements fluorescents, tels que les groupements comportant des cycles aromatiques conjugués. Les techniques de séquençage de l'ADN modernes reposent sur les interactions complémentaires entre un brin matrice, brin séquencé, et un brin en cours d'élongation. De manière générale les nucléotides modifiés utilisés pour le séquençage doivent posséder des propriétés intactes d'appariement avec leurs nucléotides complémentaires. Les nucléotides modifiés devraient conserver ces propriétés d'interactions primordiales pour leur utilisation. Or les bases azotées qui constituent les nucléotides modifiés pour le séquençage sont des analogues des bases azotées naturelles telles que l'adénine, la guanine, la thymine, l'uracile et la cytosine, et donc ne possèdent pas la même structure chimique : certains atomes sont substitués par d'autres et certains groupements sont ajoutés ou supprimés. Ces bases azotées non naturelles peuvent présenter de nombreux inconvénients comme celui par exemple de ne pas être reconnues par les organismes vivants.
Une fois incorporés, les nucléotides terminateurs sont déprotégés afin de permettre l'addition du nucléotide suivant. L'étape de déprotection fait intervenir un moyen physique ou chimique permettant de supprimer le groupement responsable de la fonction terminatrice. Les autres groupements fonctionnels associés au nucléotide modifié sont supprimés au cours d'étapes de déprotection similaires. Il faut donc généralement plusieurs étapes de déprotection au cours des différents processus de séquençage pour pouvoir passer à la détermination du nucléotide suivant. Ces différentes étapes de déprotection s'accumulent et multiplient l'emploi de réactifs puissants ou de conditions physiques extrêmes favorisant la dégradation des différentes espèces présentes dans le milieu réactionnel et notamment la dégradation des acides nucléiques. De plus, un grand nombre d'étapes de déprotection diminue considérablement la rapidité du processus et ses performances.
Encore un problème majeur rencontré lors de l'utilisation de nucléotides modifiés pour le séquençage est l'apparition de cicatrices après les étapes de déprotection. Les différentes structures chimiques servant de lien entre les groupements fonctionnels et le nucléotide sont susceptibles d'être rompus lors des étapes de déprotection. Cette rupture ne permet cependant pas de séparer l'intégralité des structures chimiques de liaison. Ainsi des parties plus ou moins importantes de ces structures restent accrochées aux nucléotides malgré les différentes étapes de déprotection. Ces résidus ont un effet très nocif sur le processus de séquençage et sur toute utilisation ou modification des acides nucléiques en général.

Quelle que soit la structure du nucléotide retenue, les nucléotides modifiés existants ne permettent pas de répondre aux attentes des méthodes de synthèses enzymatiques. Leur utilisation médiocre par les enzymes d'élongation, le placement des différents groupes fonctionnels, l'emploi systématique de bases azotées modifiées, l'obligation de conservation des interactions avec les nucléotides complémentaires, les nombreuses étapes de déprotection et la présence de cicatrices résiduelles, interdisent l'utilisation de ces nucléotides pour la synthèse enzymatique d'acide nucléique.

Gebeyehu et al., (Nucleic Acids Research, 15(11), 4513-4534) décrit une série d'analogues nucléotidiques dATP et dCTP synthétisés qui sont modifiés par l'attachement de liaisons aliphatiques contenant un groupe fonctionnel à l'amino-azote en position 6 de l'adénine ou en position 4 de la cytosine.

Kore et al., (Nucleosides, nucleotides & Nucleic acids, NUCLEOSIDES, vol. 28, no. 4, pages 292-302) décrit des cytidines 5'-monophosphate modifiées comme sondes de ligation.

Flickinger et al., (Nucleic Acids Research, Vol. 20, No. 9, 2382-2382) décrit des nucléotides biotinylés.

Petrie et al., (Bioconjugate Chem. 1991, 2, 441-446) décrit un analogue dATP qui est modifié en position 3 avec une liaison portant un fragment biotine terminal.

Beabealashvilli et al. (Biochimica et Biophysica Acta 868 (1986) 136-144) décrit des 3'-amino-2',3'-dideoxynucieoside 5'-triphosphates et leurs dérivés comme substrats de l'ADN nucléotidylexotransférase.

A ce jour, il n'existe donc pas de solution technique satisfaisante proposant des nucléotides protégés compatibles avec la synthèse d'acides nucléiques par voie enzymatique, notamment pour la synthèse enzymatique de fragments d'acide nucléique de grande longueur.

### Buts de l'invention

Un premier but de l'invention est de fournir des nucléotides modifiés adaptés à une synthèse enzymatique d'acides nucléiques.

Un autre but de l'invention est de proposer des nucléotides naturels modifiés par différents groupes fonctionnels permettant de les rendre compatibles à leur utilisation au cours d'un processus de synthèse d'acide nucléique.

Un autre but de l'invention est de fournir des nucléotides modifiés permettant de synthétiser des acides nucléiques de grande longueur, c'est à dire d'au moins plusieurs centaines ou plusieurs milliers de nucléotides, et plus particulièrement selon le procédé décrit dans la demande de brevet du même déposant non encore publiée FR 14-53455.

### Description de l'invention

La présente demande divulgue un nucléotide modifié, destiné à la synthèse-par voie enzymatique, d'acides nucléiques, tels que des acides nucléiques à longues chaînes, comprenant une base azotée « naturelle » ou un analogue de base azotée naturelle, un glucide ribose ou désoxyribose, et au moins un groupement phosphate, caractérisé en ce qu'il comprend au moins un groupement R ou R', dénommé groupement modificateur, porté :
- par ladite base azotée naturelle ou analogue
- et/ou par l'oxygène en position 3' de la molécule de ribose ou désoxyribose, permettant de bloquer la polymérisation dudit nucléotide (le groupement modificateur est alors un groupement protecteur) et/ou de permettre l'interaction dudit nucléotide avec une autre molécule, différente d'un autre nucléotide, telle qu'une protéine, lors de la synthèse des acides nucléiques, R comprenant au moins un groupement terminal fonctionnel (qui peut encore être dénommé groupement effecteur).

Plus particulièrement, le groupement modificateur n'est avantageusement pas un groupement de taille importante, tel qu'un groupement comportant des cycles aromatiques conjugués, notamment afin de permettre l'accès de l'enzyme au site réactionnel.
Le nucléotide peut être un mono-, un di- ou un triphosphate, le ou les groupement(s) phosphate étant libre(s), c'est-à-dire non modifié(s).

Le nucléotide divulgué se présente sous la forme d'une des formules (I), (III) ou (IV) suivantes : 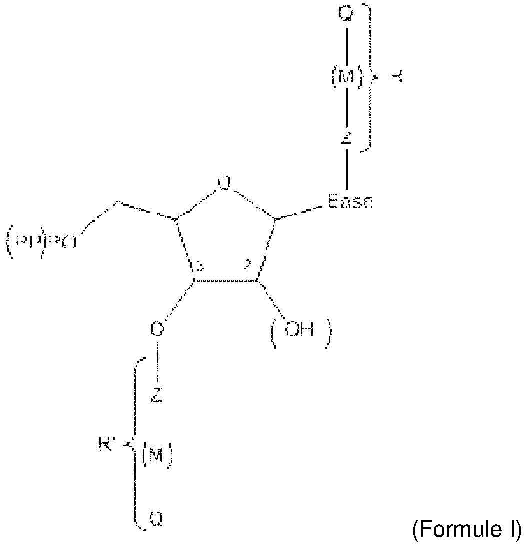 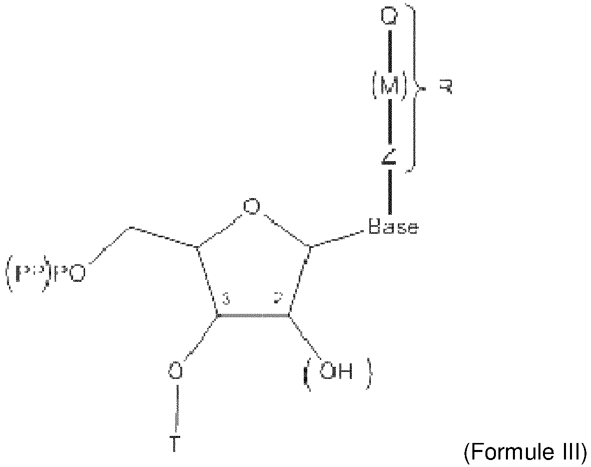 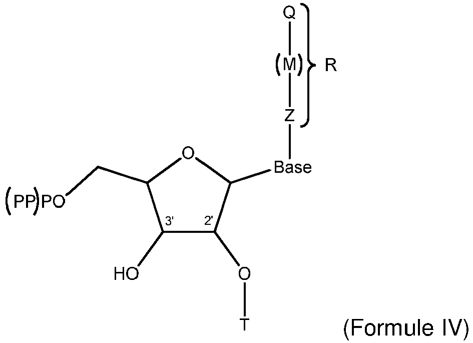 dans lesquelles :
(PP)PO représente un groupement mono-, di- ou triphosphate,
(OH) décrit la possibilité d'une molécule de ribose ou de désoxyribose,
T est un hydrogène, ou un radical clivable choisi parmi -NH₂, -N₃, -(C=O)H, -CₙH₂ₙ₊₁ avec n compris entre 1 et 30, de préférence compris entre 1 et 12, -triméthylsilyle, - phosphate, -SO₃, -(C=O)OCₙH₂ₙ₊₁ avec n compris entre 1 et 30, de préférence compris entre 1 et 12, -(C=O)SCₙH₂ₙ₊₁ avec n compris entre 1 et 30, de préférence compris entre 1 et 12 , -nitrobenzène, -benzyle, -halogènobenzyle, -amide, -carbonate, -benzoyle, -peroxyle, -nitrile, -thiol, -imide, -carbamate, -cyanate, -alcyne, -phényle, - halogènophényle, -picolyl,
M, éventuellement présent, est un groupement lié de manière covalente à Q et à Z, M étant choisi parmi alkyl, alcényl, alcyne, aryl, alkylaryl, hétéroaryl, acyl, alkyloxy, alkylamino, alkoxyamino, amido, alkylimido, alkenylimido, arylimido, fluoroalkyl, alkylphosphate, alkylthio, thioacyl, alkylsulfonyl, arylsulfonyl, alkylsulfinyl, alkylammonium, alkylsulfonium, alkylsilyl, alkylcarbonyl, alkylcarbonyl, alkylcarbanyl, alkylcarbamoyl ou alkylhydroxylamino,
Z est un groupement clivable, choisi parmi -O-,-S-, =SH-, =S=, =S-, -SiH₂-, =SiH-, =Si=, =Si-, -Se-, =SeH-, ≡Se-, =Se=, -SeH2-, -PH-, =P-, =PH=, ≡P=, ≡PH-, -PH₃-, -AsH-, =As-, =AsH=, ≡As=, ≡AsH-, -ASH₃-, amine, ester, silyl, alkyl, benzyl, nitrobenzyl, amide, carbonate, benzoyle, peroxyl, nitrile, thiol, imide, carbamate, cyanate, hydroxylamine, sulfoxyde, sulfonate, thiosulfinate, thioester, halogénure d'acyle, hypoiodyl, alcyne, halogèno-phényl, halobenzyl, picolyl, diol ou disulfure, ou choisi parmi -CH₂ ou -NH- quand M est un -nitrobenzyl-, un -nitrotolyl-, un -nitroxylyl-, un - nitronaphtyl- ou un -nitrophényl-,
Q est un groupement fonctionnel, ou effecteur, terminal du groupement R ou R', Q étant choisi parmi la biotine, une protéine, un polynucléotide de séquence définie, un glucide, un antigène, une hormone, un neurotransmetteur, un glycoside tel que la digoxine, un radical sulfuré, en particulier portant une fonction thiol tel que le glutathion, ou un ligand bidentate tel que le catéchol,
R et R' pouvant être présents indépendamment ou simultanément, et lorsque R et R' sont présents simultanément :
   les groupements Z peuvent être identiques ou différents,
   les groupements M peuvent être identiques ou différents,
   les groupements Q peuvent être identiques ou différents.
   "base" représente une base azotée "naturelle" choisie parmi l'adénine, la thymine, la cytosine, la guanine ou l'uracile ou un analogue de base azotée naturelle, à l'exception de la thymine quand R' est présent et Q comprend la biotine.

De manière préférée, T, lorsqu'il n'est pas hydrogène, ainsi que R et R' constituent des groupements assurant la terminaison de chaîne de l'étape d'élongation d'un processus de synthèse d'acides nucléiques.

Par radical ou groupement clivable, on entend un radical ou un groupement lié de manière covalente à l'oxygène en position 3' ou 2' de la molécule de ribose ou en position 3' de la molécule de désoxyribose, ou à un atome de la base azotée, ladite liaison pouvant être rompue par voie chimique ou photochimique. De manière avantageuse, la rupture de l'ensemble des liaisons des radicaux ou groupements clivables T et Z de la molécule de nucléotide s'effectue intégralement et simultanément, c'est-à-dire au cours de la même étape de "déprotection", en particulier par l'application d'une même condition ou par l'action conjointe d'un même réactif.

Cette suppression des groupes modificateurs est de préférence totale pour aboutir à la génération de nucléotide exempt de tout groupe modificateur, c'est-à-dire identique à un nucléotide naturel (à la structure de la base azotée près).

Comme indiqué précédemment, les groupements R et R' sont des groupements assurant avantageusement la terminaison de chaîne de l'étape d'élongation d'un procédé de synthèse d'acides nucléiques. Ces groupements R et R' peuvent posséder des propriétés, au moyen du groupement fonctionnel Q placé à l'extrémité libre de R et R', d'accrochage à une autre molécule, différente d'un acide nucléique, par exemple une molécule présente sur un support.

En effet, au cours du processus de synthèse d'acides nucléiques, par exemple dans le procédé décrit dans FR 14-53455 cité plus haut, certaines étapes supposent que les nucléotides modifiés employés puissent interagir avec des supports solides. Ces supports solides comportent à leur surface des molécules, protéines ou fonctions chimiques compatibles avec les groupements modificateurs des présents nucléotides. Cette fonctionnalité des nucléotides modifiés est alors primordiale pour le bon déroulement du processus de synthèse d'acides nucléiques. Dans un mode de réalisation préféré, les nucléotides modifiés comportent un groupement qui leur permet de s'associer à un support solide afin d'être purifiés, par exemple, en formant, avec les molécules présentes à la surface d'un support solide, des complexes d'association ayant une constante de dissociation très faible, en particulier inférieure à 10⁻⁶ mol/L. A la suite de l'étape de déprotection le groupement assurant cette fonction d'association est alors susceptible d'être détruit supprimant ainsi l'interaction entre le nucléotide et le support solide. Dans ce cas, le nucléotide présente un double avantage, à savoir la présence d'un groupement permettant la purification et la capacité de destruction de ce même groupement simultanément aux autres groupements modificateurs, sur un même nucléotide.

Il est divulgué que le groupement R modificateur est porté par la base azotée et forme l'une des structures (V) suivantes : 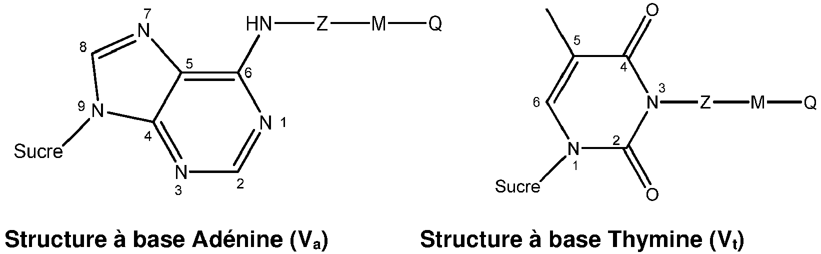 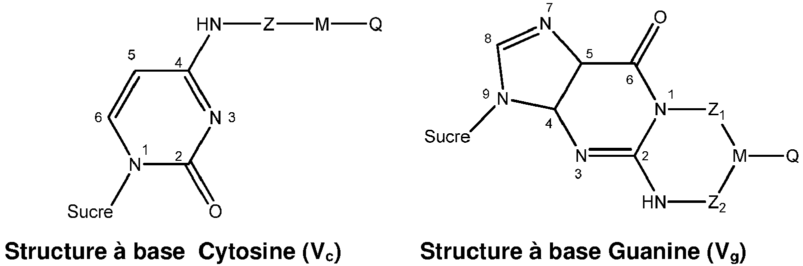 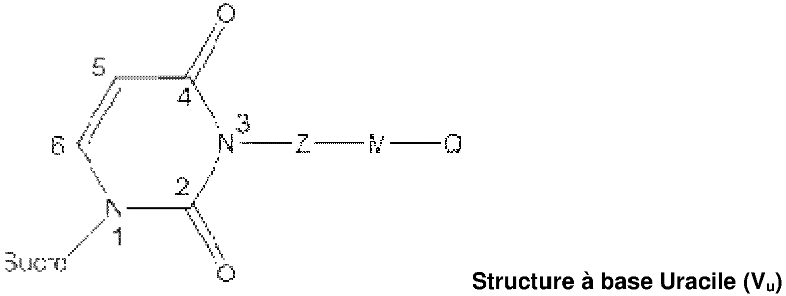 structures dans lesquelles :
« sucre » représente la liaison entre ladite base azotée et la molécule de ribose ou de désoxyribose de la molécule de nucléotide,
Z₁ et Z₂ sont des groupes Z clivables, identiques ou différents,
Les groupements Z, M et Q ont les significations décrites précédemment.

Dans ce mode de réalisation les nucléotides sont modifiés par des groupements portés par les atomes des bases azotées habituellement impliqués dans les mécanismes d'appariement Watson-Crick, c'est-à-dire portés par les atomes d'azote des fonctions amines normalement impliquées dans l'appariement à un nucléotide complémentaire. L'accrochage des différents groupements modificateurs aux atomes constituants les bases azotées est toujours réalisé par l'intermédiaire des groupements Z, de type clivables, par une liaison de type covalente.

Dans le cas de base azotée de type adénine, un mode de réalisation consiste à lier le groupement modificateur au groupe amine primaire 6-NH₂ (structure Vₐ).

Dans le cas de base azotée de type thymine, un autre mode de réalisation consiste à lier le groupement modificateur au groupe amine secondaire 3-NH (structure Vₜ).

Dans le cas de base azotée de type cytosine, un autre mode de réalisation consiste à lier le groupement modificateur au groupe amine primaire 4-NH₂ (structure V_{c}).

Dans le cas de base azotée de type uracile, un autre mode de réalisation consiste à lier le groupement modificateur au groupe amine secondaire 3-NH (structure Vᵤ). Dans le cas de base azotée de type guanine, un autre mode de réalisation consiste à lier le groupement modificateur à l'un des deux, ou simultanément aux deux, groupes amine, l'un secondaire 1-NH et l'autre primaire 2-NH₂, par l'intermédiaire de groupements clivables (Structure V_{g}). Dans le cas particulier ou les deux groupes amine 1-NH et 2-NH₂ sont simultanément utilisés pour porter les groupements modificateurs un cycle, préférentiellement composé de 6 atomes, peut apparaître entre les différents sous-groupes séparateurs. Ce cycle entraîne éventuellement une stabilisation de la structure des groupements modificateurs.

Dans ces modes de réalisations où le groupement modificateur est porté par la base azotée, les sites 3'OH et/ou 2'OH des nucléotides sont libres, favorisant leur utilisation en tant que substrats pour les enzymes d'élongation lors de la synthèse d'acides nucléiques.

Des liaisons hydrogène intermoléculaires peuvent avoir lieu. En effet, le groupement R modificateur porté par la base azotée peut former l'une des structures (VI) suivantes : 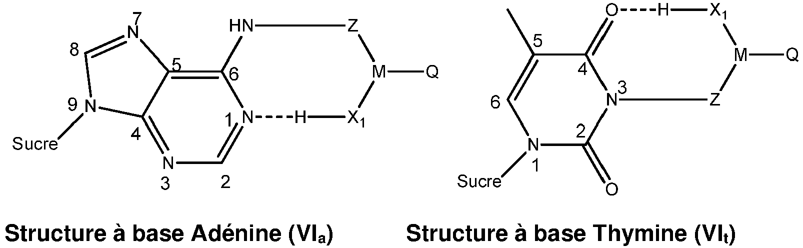 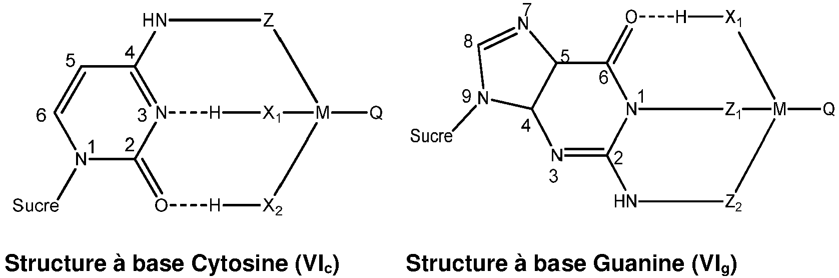 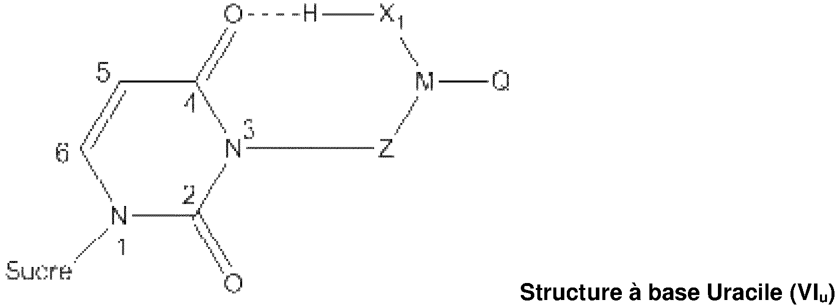 dans lesquelles :
« Sucre » représente la liaison entre ladite base azotée et la molécule de ribose ou de désoxyribose de la molécule de nucléotide,
X₁ et X₂, identiques ou différents, représentent des atomes d'azote, d'oxygène ou de soufre portés par M et aptes à former avec lesdites bases azotées du nucléotide modifié des liaisons hydrogène intramoléculaires (ces liaisons hydrogène sont alors similaires aux liaisons hydrogène intermoléculaires observées lors des appariements classiques entre nucléotides complémentaires).

Cette configuration renforce la stabilité des nucléotides modifiés. Elle influence aussi la compacité des groupements modificateurs et permet leur utilisation par des enzymes d'élongation dépendants habituellement de la présence d'un brin matrice.

La présente invention porte sur un nucléotide modifié, destiné à la synthèse d'acides nucléiques, par voie enzymatique, comprenant une base azotée « naturelle », un glucide ribose ou désoxyribose, et au moins un groupement phosphate, caractérisé en ce qu'il comprend au moins un groupement R , dénommé groupement modificateur, porté par ladite base azotée naturelle, R comprenant au moins un groupement terminal fonctionnel, ledit nucléotide se présentant sous la forme de la formule (III) suivante : 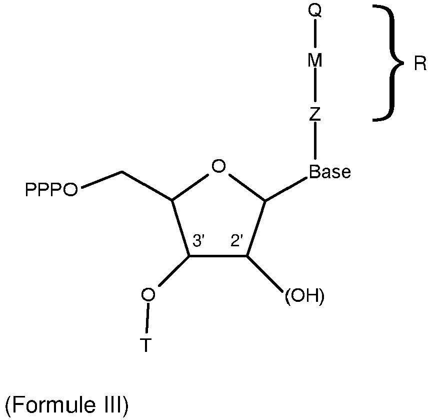 dans lesquelles :
PPPO représente un groupement triphosphate,
(OH) décrit la possibilité d'une molécule de ribose ou de désoxyribose,
T est un hydrogène, ou un radical clivable choisi parmi -NH₂, -N₃, -(C=O)H, -CₙH₂ₙ₊₁ avec n compris entre 1 et 30, de préférence compris entre 1 et 12, -triméthylsilyle, - phosphate, -SO₃, -(C=O)OCₙH₂ₙ₊₁ avec n compris entre 1 et 30, de préférence compris entre 1 et 12, -(C=O)SCₙH₂ₙ₊₁ avec n compris entre 1 et 30, de préférence compris entre 1 et 12 , -nitrobenzène, -benzyle, -halogènobenzyle, -amide, -carbonate, -benzoyle, -peroxyle, -nitrile, -thiol, -imide, -carbamate, -cyanate, -alcyne, -phényle, - halogènophényle, -picolyl,
M est un groupement lié de manière covalente à Q et à Z, M étant choisi parmi alkyl, alcényl, alcyne, aryl, alkylaryl, hétéroaryl, acyl, alkyloxy, alkylamino, alkoxyamino, amido, alkylimido, alkenylimido, arylimido, fluoroalkyl, alkylphosphate, alkylthio, thioacyl, alkylsulfonyl, arylsulfonyl, alkylsulfinyl, alkylammonium, alkylsulfonium, alkylsilyl, alkylcarbonyl, alkylcarbonyl, alkylcarbanyl, alkylcarbamoyl ou alkylhydroxylamino,
Z est un groupement clivable, choisi parmi -O-,-S-, =SH-, =S=, =S-, -SiH₂-, =SiH-, =Si=, =Si-, -Se-, =SeH-, ≡Se-, =Se=, -SeH₂-, -PH-, =P-, =PH=, ≡P=, ≡PH-, -PH₃-, -AsH-, =As-, =AsH=, ≡As=, ≡AsH-, -ASH₃-, amine, ester, silyl, alkyl, benzyl, nitrobenzyl, amide, carbonate, benzoyle, peroxyl, nitrile, thiol, imide, carbamate, cyanate, hydroxylamine, sulfoxyde, sulfonate, thiosulfinate, thioester, halogénure d'acyle, hypoiodyl, alcyne, halogèno-phényl, halobenzyl, picolyl, diol ou disulfure, ou choisi parmi -CH₂ ou -NH- quand M est un -nitrobenzyl-, un -nitrotolyl-, un -nitroxylyl-, un - nitronaphtyl- ou un -nitrophényl-,
Q est un groupement fonctionnel, ou effecteur, terminal du groupement R, Q étant choisi parmi la biotine, une protéine, un polynucléotide de séquence définie, un glucide, un antigène, une hormone, un neurotransmetteur, un glycoside tel que la digoxine, un radical sulfuré, ou un ligand bidentate tel que le catéchol,
"base" représente une base azotée "naturelle" choisie parmi l'adénine, la thymine, la cytosine, la guanine ou l'uracile,
le groupement R modificateur porté par la base azotée forme l'une des structures (VI) suivantes : 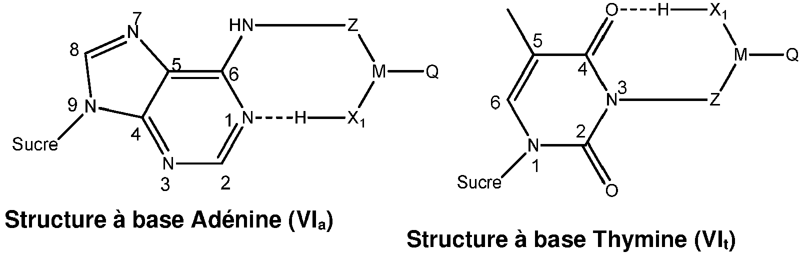 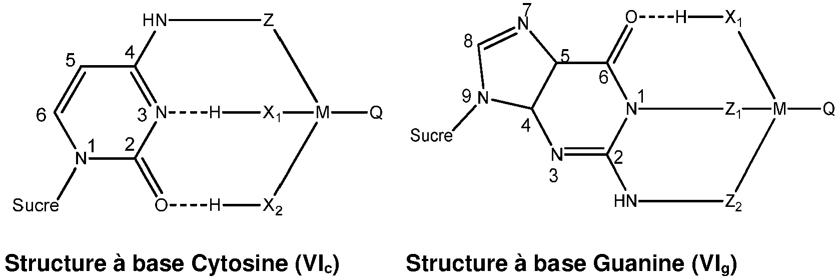 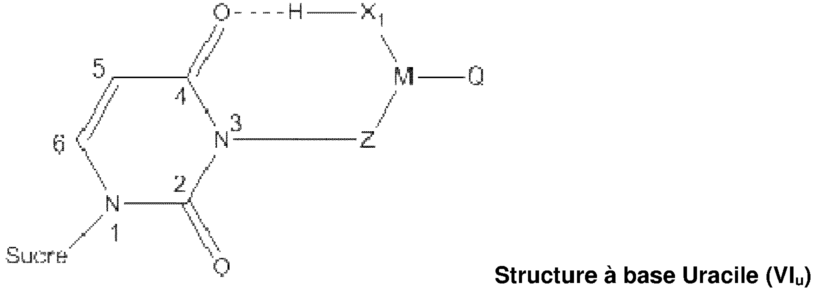 dans lesquelles :
   « Sucre » représente la liaison entre ladite base azotée et la molécule de ribose ou de désoxyribose de la molécule de nucléotide, Z₁ et Z₂ sont des groupes Z clivables, identiques ou différents,
   X₁ et X₂, identiques ou différents, représentent des atomes d'azote, d'oxygène ou de soufre portés par M et aptes à former avec lesdites bases azotées du nucléotide modifié des liaisons hydrogène intermoléculaires (similaires à celles observées lors des appariements classiques entre nucléotides complémentaires).

Dans le cas de base azotée de type adénine, un mode de réalisation préféré de la présente invention consiste à lier le groupement modificateur au groupe amine primaire 6-NH₂ (structure VIₐ). Le groupement X₁ présent favorise la création d'une liaison hydrogène intramoléculaire avec l'atome d'azote 1 du cycle purinique. Ainsi la présence d'une thymine complémentaire est mimée.

Dans le cas de base azotée de type thymine, un mode de réalisation préféré de la présente invention consiste à lier le groupement modificateur au groupe amine secondaire 3-NH (structure VIₜ). Le groupement X₁ présent favorise la création d'une liaison hydrogène intramoléculaire avec l'atome d'oxygène en position 4 du cycle pyrimidique. Ainsi la présence d'une adénine complémentaire est mimée.

Dans le cas de base azotée de type cytosine, un mode de réalisation préféré de la présente invention consiste à lier le groupement modificateur au groupe amine primaire 4-NH₂ (structure VI_{c}). Les groupements X₁ et X₂ présents favorisent la création de liaisons hydrogène intramoléculaires avec l'atome d'azote en position 3 et l'oxygène en 2 du cycle pyrimidique. Ainsi la présence d'une guanine complémentaire est mimée. Dans le cas de base azotée de type guanine, un mode de réalisation préféré de la présente invention consiste à lier le groupement modificateur aux deux groupes amines, le premier secondaire 1-NH et le second primaire 2-NH₂ (structure VI_{g}) par l'intermédiaire de groupements Z₁ et Z₂ clivables. Le groupement X₁ présent favorise la création d'une liaison hydrogène intramoléculaire avec l'atome d'oxygène en 6 du cycle purinique. Ainsi la présence d'une cytosine complémentaire est mimée.

Dans le cas de base azotée de type uracile, un mode de réalisation préféré de l'invention consiste à lier le groupement modificateur au groupe amine secondaire 3-NH (structure VIᵤ). Le groupement X₁ présent favorise la création d'une liaison hydrogène avec l'oxygène en position 4 du cycle pyrimidique. Ainsi, la présence d'une adémine complémentaire est mimée.

Les nucléotides modifiés selon la présente invention n'ont pas nécessairement vocation à s'apparier à un éventuel nucléotide complémentaire porté par un quelconque brin matrice. Lors de l'utilisation des nucléotides modifiés objets de la présente invention, pour la génération d'acides nucléiques, ces derniers ne sont pas obligatoirement destinés à être incorporés par interaction complémentaire avec un éventuel brin matrice.

Les nucléotides modifiés objets de la présente invention possèdent des caractéristiques leur permettant de perdre leurs groupements modificateurs lors d'étapes spécifiques. A l'issue de la perte de la totalité de leurs groupements modificateurs les nucléotides de la présente invention ainsi transformés recouvrent alors leur capacité à s'apparier à des nucléotides complémentaire portés par des brins matrice.

Selon un mode de réalisation particulier, le nucléotide selon l'invention est alors utilisable comme substrat de polymérases normalement dépendantes de la présence d'un brin d'acide nucléique matrice complémentaire du brin en cours de synthèse, même en absence d'un brin complémentaire.

Le radical Q terminal fonctionnel du groupement R ou R' est de préférence apte à permettre, lors de la synthèse d'acide nucléique, l'accrochage dudit nucléotide à un support solide, par l'intermédiaire d'une molécule différente d'un acide nucléique, telle qu'une protéine, fixée à la surface dudit support, et plus particulièrement apte à interagir avec des molécules autres qu'un acide nucléique, selon l'un ou l'autre des couples d'interactions suivants : antigène/anticorps, hormone/récepteur, biotine/(strept)avidine, neurotransmetteur/récepteur, polymérase/promoteur, digoxine/antidigoxine, glucide/lectine, radical sulfuré/métal tel que l'or, glutathion/glutathionne S-transférase, ou encore ligand bidentate/oxyde métallique. Les oxydes métalliques peuvent être, par exemple, TiO₂, ZrO₂, CeO₂, Fe₃O₄, Ga₂O₃, In₂O₃, Cr₂O₃, Al₂O₃, ZnO, CuO, Cu₂O₃, Mn₃O₄, Mn₂O₃, V₂O₃, MoO₂.

Les ligands bidentate peuvent être le catéchol, l'hydroxamate ou un hydroxycarboxylate.

Le radical T est dit "bloqueur" en ce qu'il protège le groupement hydroxy 3' ou le groupement hydroxy 2' du glucide contre toute addition nucléotidique supplémentaire.

De préférence, T et Z, ou Z₁, Z₂ sont clivables, lors de la synthèse d'acide nucléique, par irradiation dudit nucléotide au moyen de rayonnements électromagnétiques de longueur d'onde comprise entre 10⁻³ et 10⁻¹¹ mètre, notamment par exposition à des rayonnements ultra-violets.

La présente demande divulgue des modes de réalisation concernant les nucléotides suivants :
- un nucléotide pour lequel X₁ et X₂ sont -NH, T est -NH₂, Z est -CH₂, M est méthyl nitro benzyl-, et Q est -biotine,
- un nucléotide pour lequel X₁ et X₂ sont -NH, T est -NH₂, Z , Z₁ et Z₂ sont chacun -O-, M est -nitronaphtyl- et Q est -biotine,
- un nucléotide de formule (I) portant uniquement le groupement R' dans lequel : Z est -(C=O)-, M est -C₈H₁₆- et Q est -NH-biotinyl,
- un nucléotide de structure particulière (V) dans laquelle Z, Z₁ et Z₂ sont chacun - (COO)-, M est -ter- butylnitrobenzyl- et Q est -NH-biotinyl.

La présente invention concerne également l'utilisation de nucléotides, tels que décrits précédemment, dans un procédé de production de gènes, de séquence d'acides nucléiques synthétiques, d'ADN, d'ARN ou de polymères d'acides nucléiques, en particulier selon un procédé de synthèse enzymatique.

Une utilisation avantageuse est pour l'incorporation dudit nucléotide dans une chaîne polynucléotidique préalablement immobilisée sur un support solide, plus particulièrement lorsque la chaîne polynucléotidique est fixée par son extrémité 5' et l'incorporation dudit nucléotide est réalisée par l'extrémité 3' de la chaîne de polynucléotide.

Néanmoins, les nucléotides selon l'invention se présentent sous forme libre, en association avec un contre ion si besoin. Bien qu'ayant la capacité de se fixer à support solide, ces nucléotides sont libres de tout support au moment de leur incorporation dans la chaîne polynucléotidique. Leur structure chimique n'est donc pas liée à un support solide quelconque. La nature libre des nucléotides modifiés est particulièrement importante pour leur utilisation dans le procédé décrit dans la demande de brevet non encore publiée FR 14-53455 car les nucléotides sont ajoutés à des fragments d'acides nucléiques eux-mêmes immobilisés. Ces fragments sont libérés puis, grâce au groupement effecteur des nucléotides venant d'être incorporés, sont ensuite fixés par l'extrémité opposée à un second support solide. Ce sont donc les chaînes d'acide nucléique complètes, ou polynucléotides, de séquence désirée qui sont fixées à un support solide et non les nucléotides selon l'invention, servant à construire les polynucléotides.

La présente invention concerne également un kit comprenant au moins un nucléotide modifié selon l'invention, plus particulièrement ledit kit peut comprendre différents nucléotides modifiés, un enzyme d'élongation et un support solide apte à fixer au moins l'un des dits nucléotides.

La présente demande va être décrite plus en détail au moyen des exemples illustratifs ci-après en relation avec les figures annexées dans lesquelles :

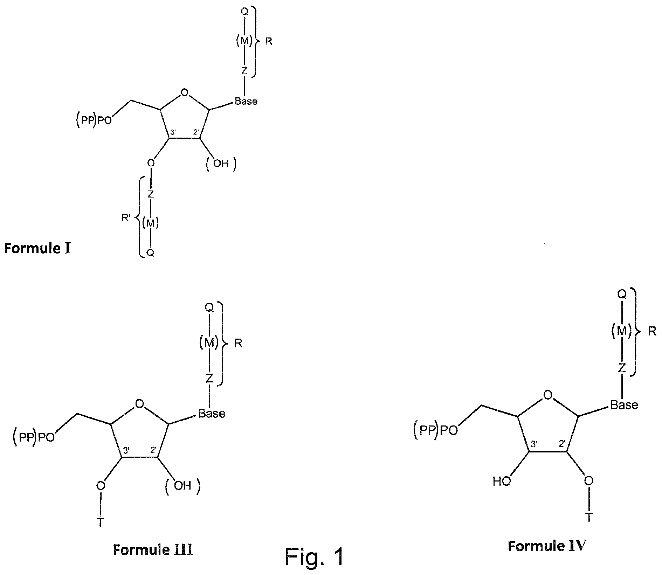

La Figure 1 montre les différentes structures générales des nucléotides modifiés objets de la présente demande ;

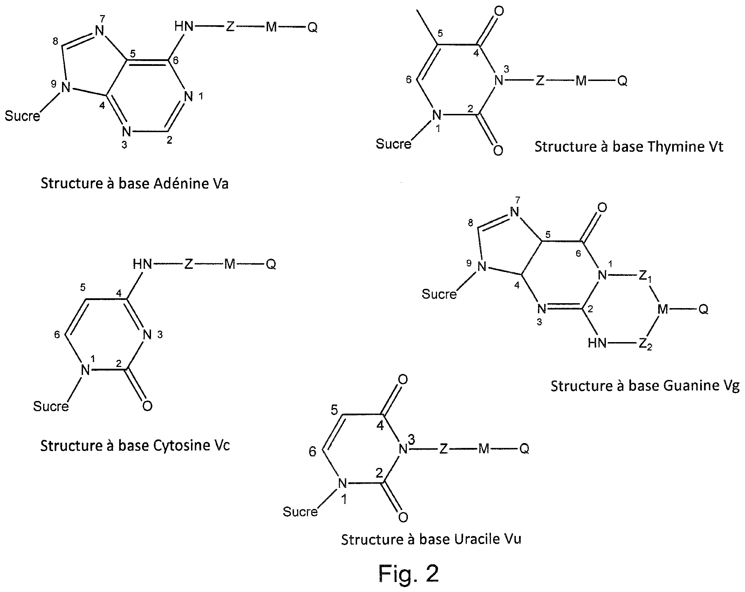

La Figure 2 présente les structures particulières des nucléotides modifiés de formule (V) ;

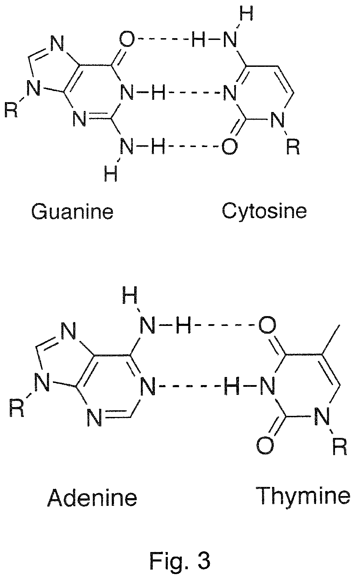

La Figure 3 présente les formules des bases azotées naturelles adénine, thymine, cytosine et guanine ;

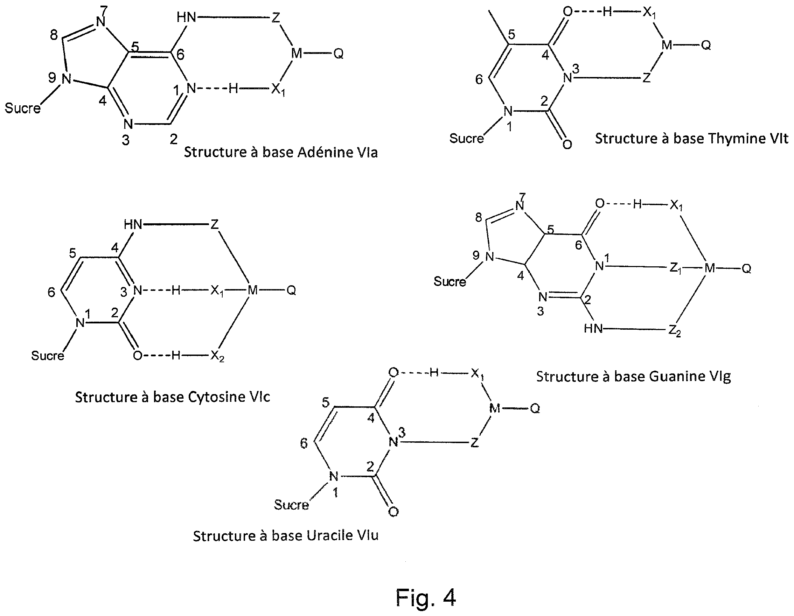

La Figure 4 montre des exemples de groupements modificateurs capables d'interactions de type liaisons hydrogène intramoléculaires, de formule (VI) ;
La Figure 5 schématise la synthèse du composé NH₂-dTTP-NitroB-Biot ;
La Figure 6 schématise la synthèse du composé NH₂-dGTP-NitroN-Biot ;
La Figure 7 schématise la synthèse des composés FA-Biot-dNTP ;
La Figure 8 schématise la synthèse du composé dATP-NitroB-Biot :
La Figure 9 schématise la synthèse du composé dCTP-NitroB-Biot ;
La Figure 10 montre un exemple de déprotection du composé NH₂-dT-NitroB-Biot polymérisé. ;
La Figure 11 montre un exemple de déprotection du composé NH₂-dG-NitroB-Biot polymérisé. ;
La Figure 12 montre un exemple de déprotection de composé FA-Biot-dNTP polymérisé ;
La Figure 13 montre un exemple de déprotection par photo-clivage du composé dA-NitroB-Biot polymérisé ;
La Figure 14 montre un exemple de déprotection par clivage chimique du composé dC-NitroB-Biot polymérisé ;
La Figure 15 présente un exemple de nucléotide modifié selon la présente demande, le groupement Q étant un catéchol ;
La Figure 16 schématise la synthèse du composé FA-Cat-dNTP.

### Exemples

### Synthèse de nucléotides modifiés (protégés) - exemples illustratifs 1 à 6

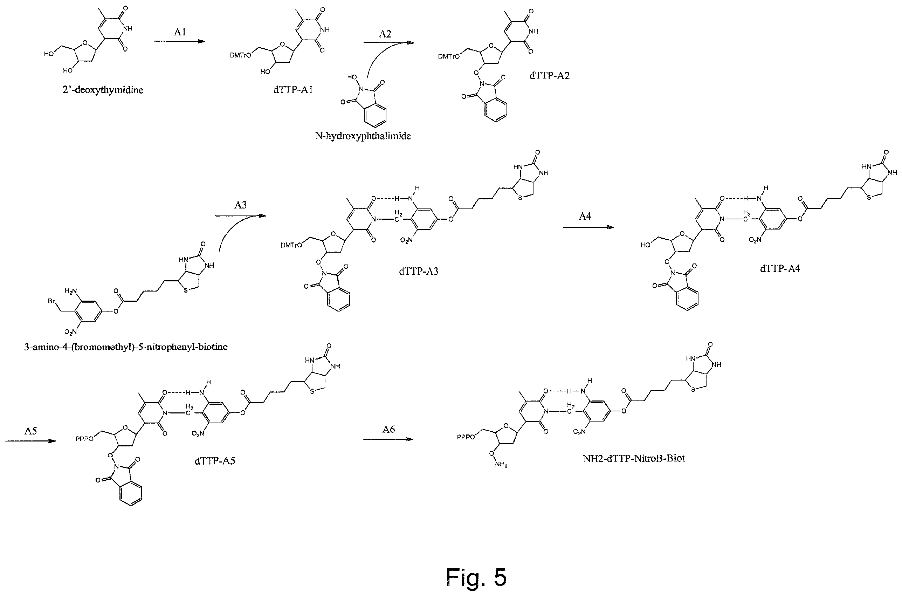

### Exemple 1 - Synthèse du composé NH₂-dTTP-NitroB-Biot (Fig 5)

Etape A1 : A 5 g de 2'-deoxythymidine solubilisés dans de la pyridine sont ajoutés 2,2 mL de Et₃N (Triéthylamine) et 175 mg de DMAP (4-diméthylaminopyridine) puis 5,25 g de DMTCl (chlorure de 4,4'-Diméthoxytrityle) à température ambiante durant la nuit. 2,4 mL de Et₃N et 1,27 mL de MsCl (Chlorure de Méthane sulfonyl) sont ensuite ajoutés au mélange. Après 2 h d'incubation à température ambiante, le mélange est filtré et lavé à l'acétate d'éthyle. Le filtrat est concentré et dissout dans 75 mL d'éthanol à quoi est ajouté 1 M de NaOH. Après chauffage à reflux pendant 1,5 h le mélange est refroidi à température ambiante et 1 M de HCl est ajouté. L'éthanol est évaporé par évaporateur rotatif et le résidu est extrait avec CH₂Cl₂. Après purification sur colonne de gel de silice le produit dTTP-A1 est obtenu.
Etape A2 : A une solution de 2,237 mmol de produit dTTP-A1, 2,1 g de triphenylphosphine et 1,3 g de N-hydroxyphthalimide dans 50 mL de tétrahydrofurane est ajouté 1,75 mL de N-N'-diisopropyl azodicarboxylate à 0°C. Après réchauffement à température ambiante durant la nuit le produit de la réaction est traité avec 0,3 mL d'eau et le solvant est évaporé sous vide. La plus grande partie des impuretés est élliminée par chromatographie donnant alors le produit dTT-A2.
Etape A3 : A un équivalent de composé dTT-A2 sont ajoutés 10 équivalents de LiH dans du DMF à température ambiante. Le mélange réagit pendant 30 min. La réaction est poursuivie par l'addition de 3-amino-4-(bromométhyl)-5-nitrophényl-biotine, puis le mélange est agité pendant plusieurs heures. Le produit dTT-A3 est obtenu.
Etape A4 : le composé dTTP-A3 est ressuspendu dans du méthanol et traité avec de l'acide chlorhydrique concentré aqueux. La solution est refroidie à -20°C durant la nuit aboutissant au produit dTTP-A4.
Etape A5 : A 425 mg de nucléoside 5'-OH analogue dTTP-A4 solubilisé dans 2 mL de pyridine et 1,7 mL de dioxane est ajouté une solution de 130 mg de 2-chloro-4H-1,2,3-benzodioxaphosphorin-4-one dans 1,3 mL de dioxane. Le mélange est laissé à température ambiante durant 20 min. Un mélange de 1,4 mmol de tributylammonium pyrophosphate dans du DMF et 3,2 mmol tributylamine est ajouté. Après 20 min une solution de 180 mg d'iode et d'eau 0,28 mL dans 14 mL de pyridine est ajoutée. Après 30 min la réaction est stoppée par l'ajout d'une solution à 5% de Na₂SO₃ aqueux. Les solvants sont évaporés sous vide. 25 mL d'eau et 20 mL de CH₃CN sont ajoutés. Le mélange est filtré et purifié par HPLC inverse pour donner un composé triphosphate, en l'occurrence dTTP-A5.
Etape A6 : 0,385 mL de méthylhydrazine froide est ajoutée à 3,725 mmol de composé dTTP-A5 dans du CH₂Cl₂ anhydre à -5°C. Après 10 min un précipité de 1,2-dihydro-4-hydroxy-2-méthyl-1-oxophthalizine est formé. Le mélange est agité durant 1 h à température ambiante. Le précipité est retiré par filtration et lavé par du CH₂Cl₂. Le filtrat est ensuite concentré sous évaporateur rotatif et purifié par chromatographie pour donner le produit NH₂-dTTP-NitroB-Biot.

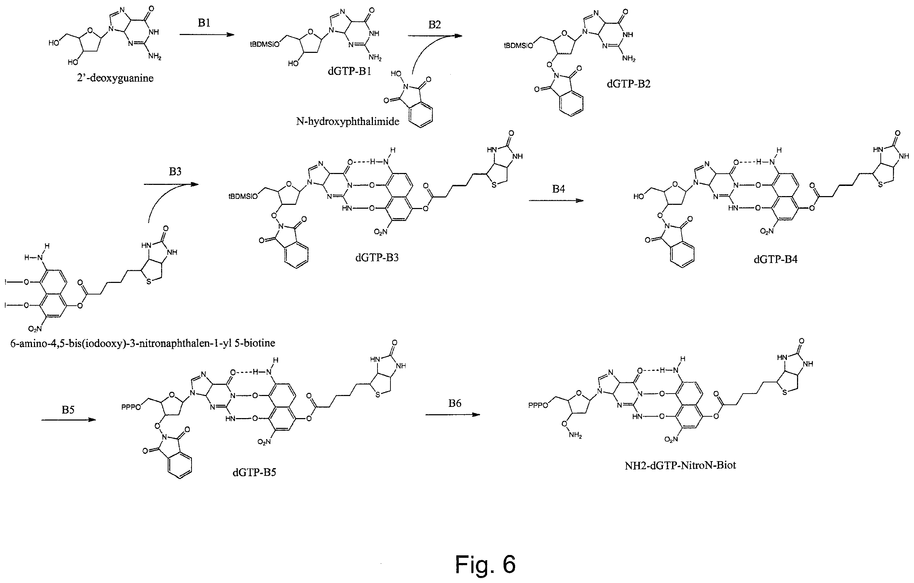

### Exemple 2 - Synthèse du composé NH₂-dGTP-NitroN-Biot (Fig 6)

Etape B1 : A une solution agitée de 1,845 mmol de 2'-déoxyguanine et de 326 mg d'imidazole dans du DMF anhydre est ajouté 2,4 mmol de chlorure de tert-butyldimethylsilyle. La réaction est incubée sous agitation à température ambiante durant 20 h. Les solvants sont éliminés sous vide et le résidu est purifié par chromatographie pour donner le produit dGTP-B1.
Etape B2 : A une solution de 2,237 mmol de produit dGTP-B1, 2,1 g de triphénylphosphine et 1,3 g de N-hydroxyphthalimide dans 50 mL de tetrahydrofurane est ajouté 1,75 mL de N-N'-diisopropyl azodicarboxylate à 0°C. Après réchauffement à température ambiante durant la nuit le produit de la réaction est traité avec 0,3 mL d'eau et le solvant est évaporé sous vide. La plus grande partie des impuretés est éliminée par chromatographie donnant alors le produit dGTP-B2.
Etape B3 : 3,785 mmol de composé dGTP-B2 sont séchées plusieurs fois par utilisation de 10 mL pyridine et évaporation sous vide. Le résidu est dissout dans 12,5 mL de CH₂Cl₂. 9 mmol de diisopropyléthylamine et 7,57 mmol de 6-amino-4,5-bis(iodooxy)-3-nitronaphthalen-1-yl 5-biotine sont ajoutés. Lorsque la réaction est complète, le mélange est dilué dans 100 mL de CH₂Cl₂, la phase organique est lavée par 50 mL de bicarbonate de sodium et 50 mL d'eau. Il est ensuite séché sur du sulfate de sodium. Les solvants sont évaporés sous vide et le produit purifié par chromatographie pour donner dGTP-B3.
Etape B4 : 3,75 mmol de composé dGTP-B3 sont dissoutes dans 20 mL de THF et traitées par 1 M de TBAF (fluorure de tetra-*n*-butylammonium) dans du THF. La réaction est complète au bout d'environ 2 h sous agitation. Le mélange est extrait au CH₂Cl₂ et purifié par chromatographie pour donner dGTP-B4.
Etape B5 : A 425 mg de nucléoside 5'-OH analogue sont traités de manière similaire à l'étape A5 de l'exemple 1. Le mélange final est filtré et purifié par HPLC inverse pour donner un composé triphosphate, en l'occurrence dGTP-B5.
Etape B6 : 0,385 mL de méthylhydrazine froide est ajoutée à 3,725 mmol de composé dGTP-B5 dans du CH₂Cl₂ anhydre à -5°C. Après 10 min un précipité de 1,2-dihydro-4-hydroxy-2-méthyl-1-oxophthalizine est formé. Le mélange est agité durant 1 h à température ambiante. Le précipité est retiré par filtration et lavé par du CH₂Cl₂. Le filtrat est ensuite concentré sous évaporateur rotatif et purifié par chromatographie pour donner le produit NH₂-dGTP-NitroN-Biot.

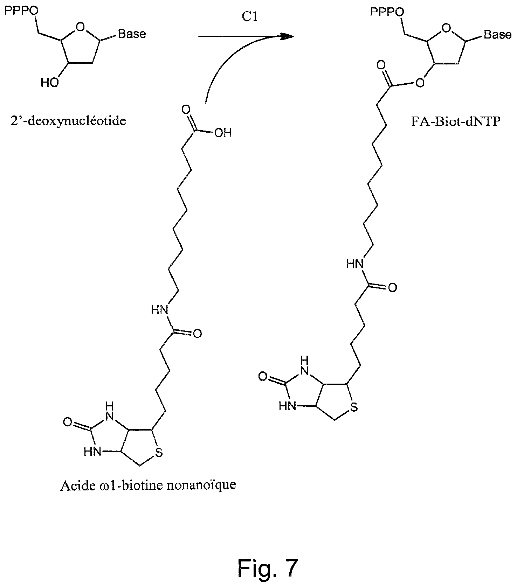

### Exemple 3 - Synthèse des composés FA-Biot-dNTP (Fig 7)

Etape C1 : 100 µL d'acide ω1-biotine nonanoïque 1 M dans du DMF sont mélangés à 100 µL de carbonyldiimidazole 1M dans du DMF. La formation d'imidazolide est réalisée en 30 s à température ambiante. Ensuite 100 µL de desoxyribonucléotides 5'-triphosphate 50 mM dans de l'eau sont ajoutés au mélange. Le produit est formé en 12 h température ambiante. Il est ensuite précipité à l'acétone et dissout dans de l'eau pour être finalement purifié par chromatographie pour donner le produit FA-Biot-dNTP.

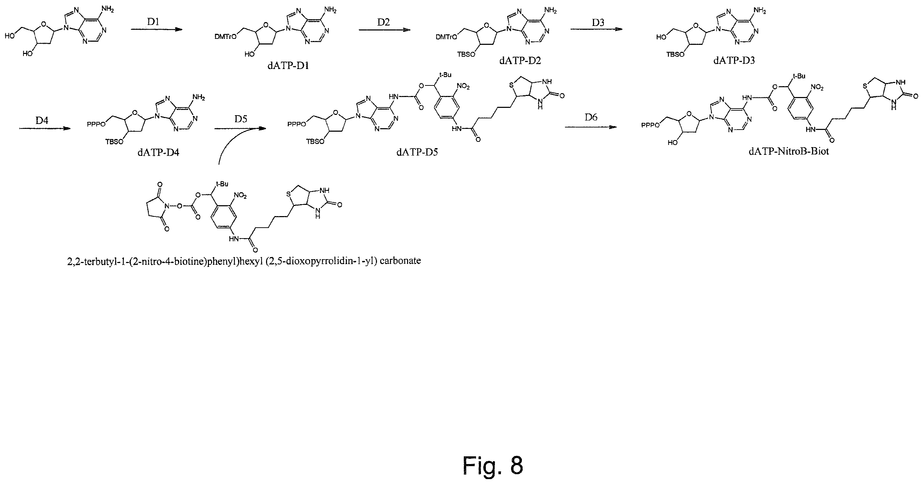

### Exemple 4 - Synthèse du composé dATP-NitroB-Biot (Fig 8)

Etape D1 : A 5 g de 2'-déoxyadénine solubilisés dans de la pyridine sont ajoutés 2,2 mL de Et₃N et 175 mg de DMAP puis 5,25 g de DMTCI à température ambiante durant la nuit. 2,4 mL de Et₃N et 1,27 mL de MsCl sont ensuite ajoutés au mélange. Après 2 h d'incubation à température ambiante, le mélange est filtré et lavé à l'acétate d'éthyle. Le filtrat est concentré et dissout dans 75 mL d'éthanol à quoi est ajouté 1 M de NaOH. Après chauffage à reflux pendant 1,5 h le mélange est refroidi à température ambiante et 1 M de HCl est ajouté. L'éthanol est évaporé par évaporateur rotatif et le résidu est extrait avec CH₂Cl₂. Après purification sur colonne de gel de silice le produit dATP-D1 est obtenu.
Etape D2 : A une solution agitée de 1,845 mmol de dATP-D1 et de 326 mg d'imidazole dans du DMF anhydre sont ajoutés 2.4 mmol de chlorure de tert-butyldimethylsilyle. La réaction est incubée sous agitation à température ambiante durant 20 h. Les solvants sont retirés par application du vide et le résidu est purifié par chromatographie pour donner le produit dATP-D2.
Etape D3 : le composé dATP-D2 est ressuspendu dans du méthanol et traité avec de l'acide chlorhydrique concentré aqueux. La solution est refroidie à -20°C durant la nuit aboutissant au produit dATP-D3.
Etape D4 : A 425 mg de nucléoside 5'-OH analogue sont traités de manière similaire à l'étape A5 de l'exemple 1. Le mélange final est filtré et purifié par HPLC inverse pour donner un composé triphosphate, en l'occurrence dATP-D4.
Etape D5 : 3,1 µmol du composé dATP-D4 dans 200 µL de NaHCO₃ 0,1 M pH 8,0 sont mélangés à 3,4 µmol de 2,2-terbutyl-1-(2-nitro-4-biotine)phényle)hexyl (2,5-dioxopyrrolidin-1-yl) carbonate dans 200 µL de dimethylformamide. La réaction est conduite à température ambiante durant toute la nuit pour donner dATP-D5 (Olejnik et al., PNAS, 1995, Vol 92, 7590-7594).
Etape D6 : 3,75 mmol du composé dATP-D5 sont dissous dans 20 mL de THF et traité par 1 M de TBAF (fluorure de tetra-*n*-butylammonium) dans du THF. La réaction est complète au bout d'environ 2 h sous agitation. Le mélange est extrait au CH₂Cl₂ et purifié par chromatographie pour donner dATP-NitroB-Biot.

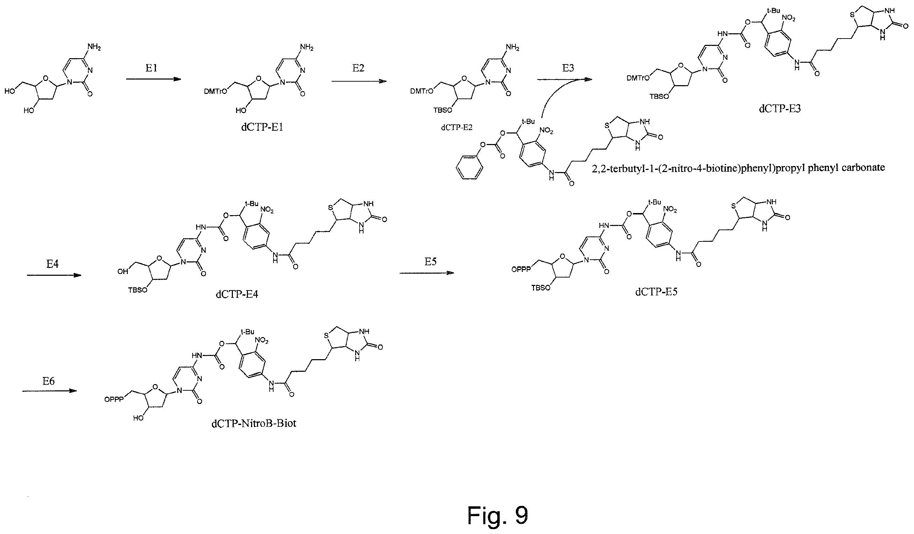

### Exemple 5 - Synthèse du composé dCTP-NitroB-Biot (Fig 9)

Etape E1 : A 5 g de 2'-deoxycytidine solubilisés dans de la pyridine sont ajoutés 2,2 mL de Et₃N et 175 mg de DMAP puis 5,25 g de DMTCI à température ambiante durant la nuit. 2,4 mL de Et₃N et 1,27 mL de MsCl sont ensuite ajoutés au mélange. Après 2 h d'incubation à température ambiante, le mélange est filtré et lavé à l'acétate d'éthyle. Le filtrat est concentré et dissout dans 75 mL d'éthanol à quoi est ajouté 1 M de NaOH. Après chauffage à reflux pendant 1,5 h le mélange est refroidit à température ambiante et 1 M de HCl est ajouté. L'éthanol est évaporé par évaporateur rotatif et le résidu est extrait avec CH₂Cl₂. Après purification sur colonne de gel de silice le produit dCTP-E1 est obtenu.
Etape E2 : A une solution agitée de 1,845 mmol de dCTP-E1 et de 326 mg d'imidazole dans du DMF anhydre est ajouté 2.4 mmol de chlorure de tert-butyldimethylsilyl. La réaction est incubée sous agitation à température ambiante durant 20 h. Les solvants sont retirés par application du vide et le résidu est purifié par chromatographie pour donner le produit dCTP-E2.
Etape E3 : Le composé dCTP-E2 est dissout dans de l'éthanol absolu et refroidit à 0°C. Une solution équimolaire de 2,2-terbutyl-1-(2-nitro-4-biotine)phényle)propyl phényle carbonate dans de l'éthanol absolu est ajoutée goute à goute. Le mélange est agité à température ambiante durant toute la nuit. La solution est filtrée, lavée à l'eau et extraite au CH₂Cl₂ pour donner dCTP-E3.
Etape E4 : le composé dCTP-E3 est ressuspendu dans du méthanol et traité avec de l'acide chlorhydrique concentré aqueux. La solution est refroidie à -20°C durant la nuit aboutissant au produit dCTP-E4.
Etape E5 : A 425 mg de nucléoside 5'-OH analogue sont traités de manière similaire à l'étape A5 de l'exemple 1. Le mélange final est filtré et purifié par HPLC inverse pour donner un composé triphosphate, en l'occurrence dCTP-E5.
Etape E6 : 3,75 mmol du composé dCTP-E5 sont dissous dans 20 mL de THF et traité par 1 M de TBAF (fluorure de tetra-n-butylammonium) dans du THF. La réaction est complète au bout d'environ 2 h sous agitation. Le mélange est extrait au CH₂Cl₂ et purifié par chromatographie pour donner dCTP-NitroB-Biot.

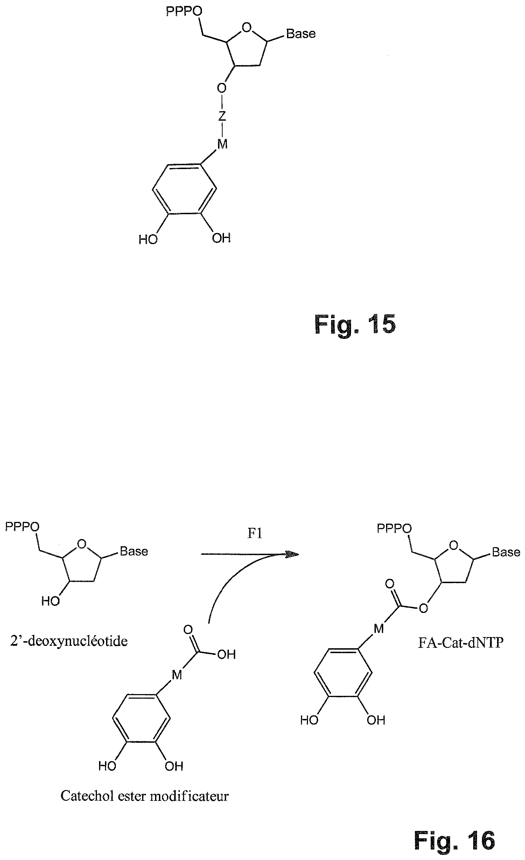

Exemple 6 - Synthèse des composés FA-Cat-dNTP (figure 16)

Etape F1 : 100 µL de catechol ester modificateur 1 M dans du DMF sont mélangés à 110 µL de dicyclohexylcarbodiimide (DCC) 1M et 5 µL de 4-(Diméthylamino)pyridine 100% dans du DMF. Le mélange est incubé à 0°C pendant 5 min. Ensuite 500 µL de désoxyribonucléotide 5'-triphosphate 50 mM dans du DMF sont ajoutés au mélange. Le produit est formé en 3 h à température ambiante. Il est ensuite précipité à l'acétone et dissous dans de l'eau pour être finalement purifié par chromatographie pour donner le produit FA-Cat-dNTP.

### Déprotection

Après l'addition du nucléotide à une chaîne d'acide nucléique, les exemples suivants 7 à 11 illustrent des modes de réalisation de la déprotection dudit nucléotide, c'est-à-dire l'élimination du ou des groupement(s) modificateur(s).

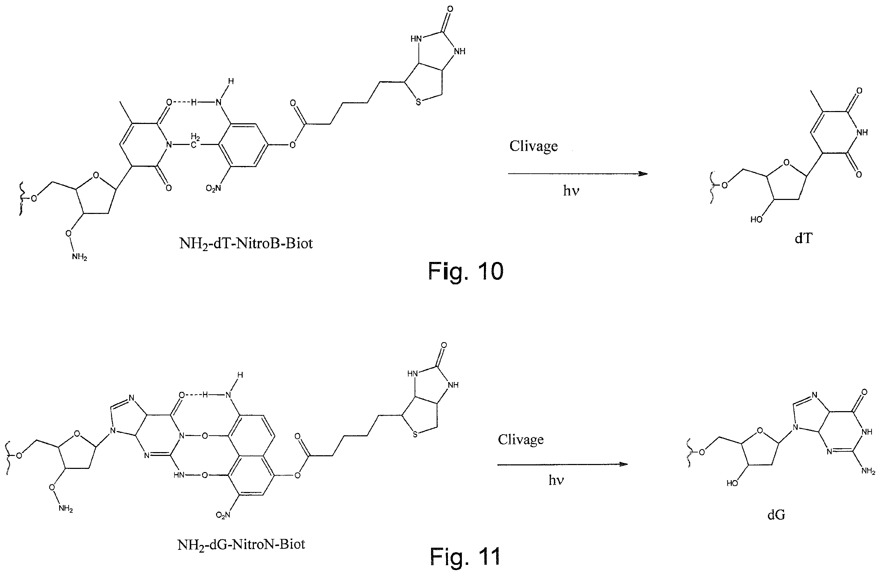

### Exemple 7 - Déprotection des nucléotides polymérisés NH₂-dT-NitroB-Biot (Fig 10)

Le clivage des différents groupements modificateurs est réalisé simultanément lors du processus suivant. 20mM de composé NH₂-dT-NitroB-Biot en solution aqueuse sont traités avec une solution comprenant 350 à 700 mM de NaNO₂ et 1 M NaOAc pH 5,5. Après 1 à 2 min d'incubation à température ambiante exposé sous lumière UV de longueur d'onde 365 nm, la réaction est stoppée par l'addition de tampon phosphate 1 M pH 7,0 et l'arrêt de l'illumination. Le produit de la réaction de déprotection est dT.

### Exemple 8 - Déprotection des nucléotides polymérisés NH₂-dG-NitroN-Biot (Fig 11)

Le clivage des différents groupements modificateurs est réalisé simultanément lors du processus suivant : 20mM de composé NH₂-dG-NitroN-Biot en solution aqueuse sont traités avec une solution comprenant 350 à 700 mM de NaNO₂ et 1 M NaOAc pH 5,5. Après 1 à 2 min d'incubation à température ambiante exposé sous lumière UV de longueur d'onde 365 nm, la réaction est stoppée par l'addition de tampon phosphate 1 M pH 7,0 et l'arrêt de l'illumination. Le produit de la réaction de déprotection est dG.

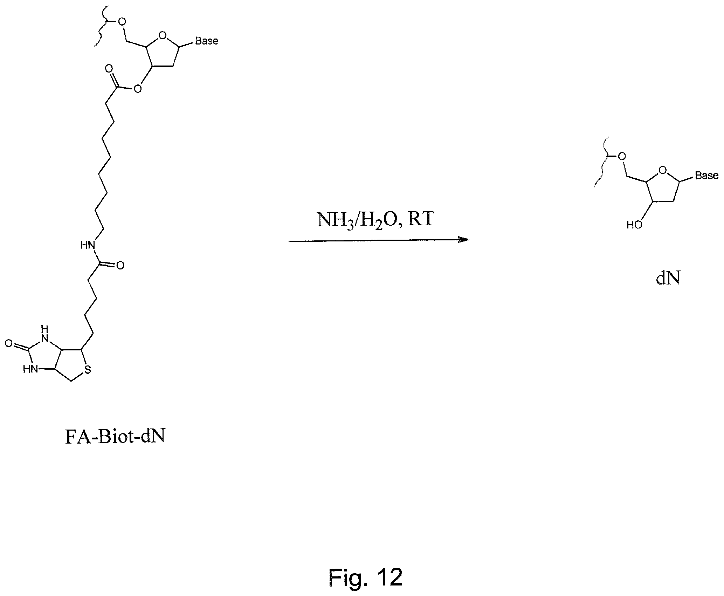

### Exemple 9 - Déprotection des nucléotides polymérisés de type FA-Biot-dN (Fig 12)

Le clivage des groupements modificateurs portés par l'extrémité 3'-OH est réalisé par hydrolyse de la fonction ester par une solution aqueuse d'ammoniaque, 1 à 100 mM, à température ambiante pendant 1 h. Le produit obtenu est de type dN.

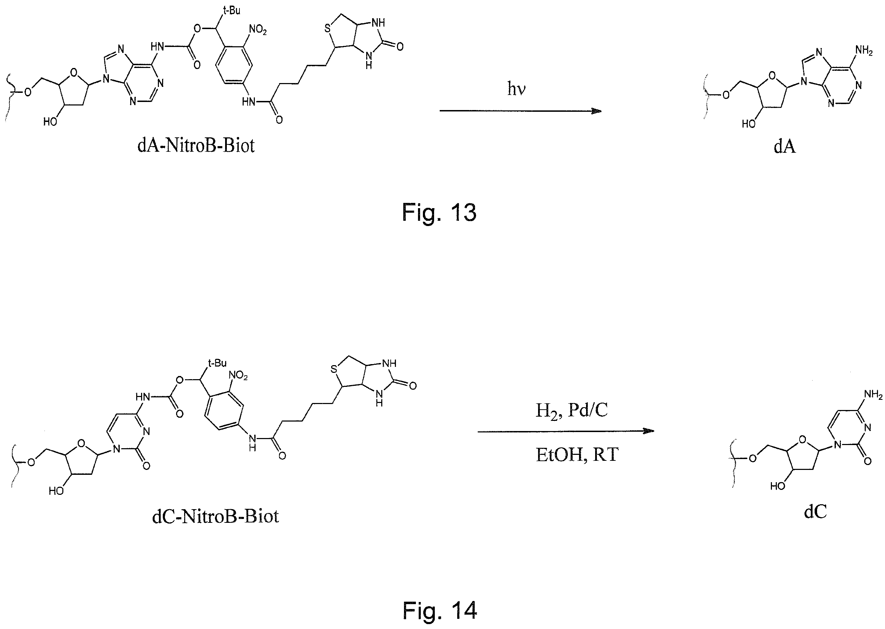

### Exemple 10 - Déprotection des nucléotides polymérisés dA-NitroB-Biot par photo-clivage (Fig 13)

Le clivage des groupements modificateurs portés au niveau de la base azotée est réalisé par photo-clivage. Le composé dA-NitroB-Biot est exposé à une source d'UV de longueur d'onde de 300 à 370 nm à température ambiante. La source d'UV est arrêtée après 30 à 300 secondes pour donner le produit dA.

### Exemple 11 - Déprotection des nucléotides polymérisés dC-NitroB-Biot par clivage chimique (Fig 14)

Le clivage des groupements modificateurs portés au niveau de la base azotée est réalisé par clivage chimique. 0,01 mmol de composé dC-NitroB-Biot est dissout dans 0,1 mL d'éthanol. Une solution de 0,02 mmol de sodium de tetrachloropalladate II dissout dans de l'éthanol est ajoutée. Du dihydrogène gazeux est bullé dans le mélange sous agitation pendant 20min. Le produit obtenu est dC.
Une procédure similaire est décrite par Kobayashi et al (Science, 2004, 304, 1305-1308) utilisant du palladium immobilisé et un flux de H₂ de 1 mL/min dans du THF peut être utilisée en variante.

### Exemple 12 - Utilisation de nucléotides selon l'invention pour une synthèse d'acides nucléiques

Les nucléotides modifiés objet de la présente invention peuvent être avantageusement utilisés pour réaliser la synthèse enzymatique d'acides nucléiques sans présence de brins matrices selon le procédé décrit dans la demande de brevet FR 14-53455. L'enzyme choisie pour la réalisation de l'étape d'addition des nucléotides modifiés est la terminale déoxynucléotidyl transférase ou TdT disponible commercialement.

L'amorce utilisée pour initier la synthèse est donnée ci-dessous:

| | |
|---|---|
| 5'-AGCCAAGCGGTCGCGATGAT-3' | Seq N°1 |

Les nucléotides modifiés utilisés sont NH₂-dTTP-NitroB-Biot préparés selon l'exemple 1. Ils permettent d'ajouter un T à la séquence N°1 présentée. Il est attendu qu'un seul et unique nucléotide sera ajouté à chaque fragment d'ADN au cours de chaque étape d'élongation, tel que décrit ci-après.

Une plaque de verre portant des fragments « de capture » ayant la séquence suivante: 5'-GTCCGCTTGGCT-3' Seq N°2
fixés par leur extrémité 3' à cette plaque de verre, est utilisée pour capturer les amorces de séquence 1. Cette plaque en verre constitue le fond d'une chambre réactionnelle paralélipipédique d'un volume de 50 µL. La capture est réalisée en utilisant une solution tampon comprenant: 20 mM Tris-HCI (pH=7,5), 500 mM LiCI et 1 mM EDTA dans laquelle sont ajoutés 2 pmol d'amorce. L'étape de capture est réalisée en 30 min à température ambiante. Une fois les amorces capturées, la plaque est lavée par l'ajout et l'élimination de 3 fois 50µL de la solution tampon suivante : 20 mM Tris-HCI (pH=7,5), 200 mM LiCI et 1 mM EDTA.

La synthèse démarre par l'ajout des réactifs suivants dans la chambre réactionnelle: 50 U de TdT, 1 M de cacodylate de potassium, 125 mM de Tris-HCl, 0,05% (v/v) de Triton X-100, 5 mM de CoCl2, à pH 7,2. Sont ensuite ajoutés 100 µM de nucléotides NH2-dTTP-NitroB-Biot libres et en solution avec leur contre ions. L'enzyme à 2µM est finalement ajoutée pour démarrer la réaction d'addition. Le volume total de réaction est de 50 µL. Le mélange est incubé pendant 5 min à 37°C.

Une fois la réaction de synthèse achevée, la plaque est lavée 3 fois avec la solution tampon suivante : 20 mM Tris-HCI (pH=7,5), 200 mM LiCI et 1 mM EDTA.
Cela a pour effet d'assurer que les nucléotides NH2-dTTP-NitroB-Biot introduits en excès sont éliminés laissant la chambre réactionnelle et le support solide vierge de tout nucléotide n'ayant pas réagi. A la fin du lavage, 50µL d'un tampon 20 mM Tris-HCI (pH=7,5) sont ajoutés dans la chambre réactionnelle et la température est élevée jusqu'à 90°C. Cela a pour effet d'assurer le décrochage des fragments ayant incorporé le nucléotide modifié NH2-dTTP-NitroB-Biot. Ces fragments sont collectés et transférés dans un tube épendorf neuf.

Les fragments d'ADN ayant incorporés le nucléotide protégé NH₂-dTTP-NitroB-Biot sont ensuite purifiés selon la procédure suivante. Des billes magnétiques revêtues de streptavidine commerciales (ThermoScientific) préparées selon le protocole du fabriquant sont ajoutées aux 50 µL du mélange réactionnel précédent. Après 1 h d'incubation à température ambiante les billes magnétiques sont collectées grâce à un aimant adapté. Le surnageant est ensuite retiré. Les billes sont ensuite lavées 3 fois avec le tampon de lavage: tampon tris pH 7,2 avec 0,1% de Tween-20.

Les billes magnétiques auxquelles se sont fixés les fragments d'ADN ayant incorporé les nucléotides modifiés NH₂-dTTP-NitroB-Biot sont re-suspendues dans une solution comprenant 350 à 700 mM de NaNO₂ et 1 M NaOAc à pH 5,5. Le mélange est incubé durant 1 à 2 min à température ambiante sous exposition aux UV (365 nm). La réaction est stoppée par l'addition de tampon phosphate 1 M à pH 7,0 et l'arrêt de l'illumination. Cette opération permet le "décrochage" des fragments d'ADN de leurs supports (billes).

Les billes magnétiques sont collectées grâce à un aimant adapté. Le surnageant est récupéré et analysé par gel d'électrophorèse et spectromètre MALDI-TOF MS pour vérifier la bonne incorporation de la base T au niveau de l'extrémité 3' de la séquence N°1 dans plus de 99% des cas.

Une nouvelle étape d'élongation peut ensuite être réalisée si nécessaire selon le même protocole.

### Exemple 13 - Autre exemple d'utilisation de nucléotides selon l'invention pour une synthèse d'acides nucléiques

Les nucléotides modifiés objet de la présente invention peuvent être avantageusement utilisés pour réaliser la synthèse enzymatique d'acides nucléiques sans présence de brins matrices selon le procédé décrit dans la demande de brevet FR 14-53455. L'enzyme choisie pour la réalisation de l'étape d'addition des nucléotides modifiés est la terminale déoxynucléotidyl transférase ou TdT disponible commercialement.

L'amorce utilisée pour initier la synthèse est donnée ci-dessous: 5'-AGCCAAGCGGTCGCGATGAT-3' Seq N°1

Les nucléotides modifiés utilisés sont NH2-dGTP-NitroB-Biot préparé selon l'exemple 2. Ils permettent d'ajouter un G à la séquence N°1 présentée. Il est attendu qu'un seul et unique nucléotide sera ajouté à chaque fragment d'ADN au cours de chaque étape d'élongation, tel que décrit ci-après.

Une plaque de verre présentant des fragments de capture ayant la séquence suivante: 5'-GTCCGCTTGGCT-3' Seq N°2
et fixés par leur extrémité 3' à cette plaque de verre, est utilisée pour capturer les amorces de séquence 1. Cette plaque en verre constitue le fond d'une chambre réactionnelle paralélipipédique d'un volume de 50 µL. La capture est réalisée en utilisant une solution tampon comprenant: 20 mM Tris-HCI (pH=7,5), 500 mM LiCI et 1 mM EDTA dans laquelle sont ajoutés 2 pmol d'amorce. L'étape de capture est réalisée en 30 min à température ambiante. Une fois les amorces capturées, la plaque est lavée par l'ajout et l'élimination de 3 fois 50µL de la solution tampon suivante : 20 mM Tris-HCI (pH=7,5), 200 mM LiCl et 1 mM EDTA.

La synthèse démarre par l'ajout des réactifs suivants dans la chambre réactionnelle: 50 U de TdT, 1 M de cacodylate de potassium, 125 mM de Tris-HCl, 0.05% (v/v) de Triton X-100, 5 mM de CoCl2, pH 7,2. Sont ensuite ajoutés 100 µM de nucléotides NH2-dGTP-NitroB-Biot libre et en solution avec leur contre ions. L'enzyme à 2µM est finalement ajoutée pour démarrer la réaction d'addition. Le volume total de réaction est de 50 µL. Le mélange est incubé pendant 5 min à 37°C.

Une fois la réaction de synthèse achevée, la plaque est lavée 3 fois avec la solution tampon suivante : 20 mM Tris-HCI (pH=7,5), 200 mM LiCl et 1 mM EDTA. Cela a pour effet d'assurer que les nucléotides NH2-dGTP-NitroB-Biot introduits en excès sont éliminés laissant la chambre réactionnelle et le support solide vierge de tout nucléotide n'ayant pas réagit. A la fin du lavage, 50µL d'un tampon 20 mM Tris-HCI (pH=7,5) sont ajoutés dans la chambre réactionnelle et la température est élevée jusqu'à 90°C. Cela a pour effet d'assurer le décrochage des fragments ayant incorporé le nucléotide modifié NH2-dGTP-NitroB-Biot. Ces fragments sont collectés et transférés dans un tube épendorf neuf.

Les fragments d'ADN ayant incorporé le nucléotide protégé NH2-dGTP-NitroB-Biot sont ensuite purifiés selon la procédure suivante. Des billes magnétiques revêtues de streptavidine commerciales (ThermoScientific) préparées selon le protocole du fabriquant sont ajoutées aux 50 µL du mélange réactionnel précédent. Après 1 h d'incubation à température ambiante les billes magnétiques sont collectées grâce à un aimant adapté. Le surnageant est ensuite retiré. Les billes sont ensuite lavées 3 fois avec la solution tampon de lavage: tampon tris pH 7,2 avec 0,1% de Tween-20.

Les billes magnétiques auxquelles se sont fixés les fragments d'ADN ayant incorporé les nucléotides modifiés NH2-dGTP-NitroB-Biot sont re-suspendues dans une solution comprenant 350 à 700 mM de et 1 M NaOAc à pH 5,5. Le mélange est incubé durant 1 à 2 min à température ambiante sous exposition aux UV (365nm). La réaction est stoppée par l'addition de tampon phosphate 1 M pH 7,0 et l'arrêt de l'illumination. Cette opération permet le "décrochage" des fragments d'ADN de leurs supports (billes).

Les billes magnétiques sont collectées grâce à un aimant adapté. Le surnageant est récupéré et analysé par gel d'électrophorèse et spectromètre MALDI-TOF MS pour vérifier la bonne incorporation de la base T au niveau de l'extrémité 3' de la séquence N°1 dans plus de 99% des cas.

Si nécessaire, une nouvelle étape d'élongation peut ensuite être réalisée selon le même protocole.

### Application industrielle

Les nucléotides modifiés objets de la présente invention améliorent les performances de procédés de synthèse d'acides nucléiques en permettant notamment la synthèse d'acides nucléiques de grande longueur de très grande qualité. Ces nucléotides peuvent être utilisés pour la production, à plus ou moins grande échelle, de gènes ou séquences d'acides nucléiques synthétiques. Ces nucléotides modifiés sont particulièrement destinés à la synthèse d'acides nucléiques tels que l'ADN ou ARN à des fins de recherche, de développement ou d'industrialisation dans le domaine de la biotechnologie ou plus généralement dans le domaine large de la biologie.

## Revendications

1. Nucléotide modifié, destiné à la synthèse d'acides nucléiques, par voie enzymatique, comprenant une base azotée « naturelle », un glucide ribose ou désoxyribose, et au moins un groupement phosphate,
**caractérisé en ce qu'**il comprend au moins un groupement R , dénommé groupement modificateur, porté par ladite base azotée naturelle, R comprenant au moins un groupement terminal fonctionnel, ledit nucléotide se présentant sous la forme de la formule (III) suivante : 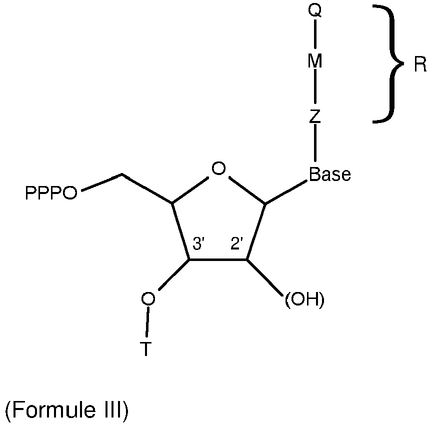 dans lesquelles :
PPPO représente un groupement triphosphate,
(OH) décrit la possibilité d'une molécule de ribose ou de désoxyribose,
T est un hydrogène, ou un radical clivable choisi parmi -NH₂, -N₃, -(C=O)H, -CₙH₂ₙ₊₁ avec n compris entre 1 et 30, de préférence compris entre 1 et 12, -triméthylsilyle, - phosphate, -SO₃, -(C=O)OCₙH₂ₙ₊₁ avec n compris entre 1 et 30, de préférence compris entre 1 et 12, -(C=O)SCₙH₂ₙ₊₁ avec n compris entre 1 et 30, de préférence compris entre 1 et 12 , -nitrobenzène, -benzyle, -halogènobenzyle, -amide, -carbonate, -benzoyle, -peroxyle, -nitrile, -thiol, -imide, -carbamate, -cyanate, -alcyne, -phényle, - halogènophényle, -picolyl,
M est un groupement lié de manière covalente à Q et à Z, M étant choisi parmi alkyl, alcényl, alcyne, aryl, alkylaryl, hétéroaryl, acyl, alkyloxy, alkylamino, alkoxyamino, amido, alkylimido, alkenylimido, arylimido, fluoroalkyl, alkylphosphate, alkylthio, thioacyl, alkylsulfonyl, arylsulfonyl, alkylsulfinyl, alkylammonium, alkylsulfonium, alkylsilyl, alkylcarbonyl, alkylcarbonyl, alkylcarbanyl, alkylcarbamoyl ou alkylhydroxylamino,
Z est un groupement clivable, choisi parmi -O-,-S-, =SH-, =S=, =S-, -SiH₂-, =SiH-, =Si=, =Si-, -Se-, =SeH-, ≡Se-, =Se=, -SeH2-, -PH-, =P-, =PH=, =P=, ≡PH-, -PH₃-, -AsH-, =As-, =AsH=, ≡As=, ≡AsH-, -ASH₃-, amine, ester, silyl, alkyl, benzyl, nitrobenzyl, amide, carbonate, benzoyle, peroxyl, nitrile, thiol, imide, carbamate, cyanate, hydroxylamine, sulfoxyde, sulfonate, thiosulfinate, thioester, halogénure d'acyle, hypoiodyl, alcyne, halogèno-phényl, halobenzyl, picolyl, diol ou disulfure, ou choisi parmi -CH₂ ou -NH- quand M est un -nitrobenzyl-, un -nitrotolyl-, un -nitroxylyl-, un - nitronaphtyl- ou un -nitrophényl-,
Q est un groupement fonctionnel, ou effecteur, terminal du groupement R, Q étant choisi parmi la biotine, une protéine, un polynucléotide de séquence définie, un glucide, un antigène, une hormone, un neurotransmetteur, un glycoside tel que la digoxine, un radical sulfuré, ou un ligand bidentate tel que le catéchol,
"base" représente une base azotée "naturelle" choisie parmi l'adénine, la thymine, la cytosine, la guanine ou l'uracile,
le groupement R modificateur porté par la base azotée forme l'une des structures (VI) suivantes : 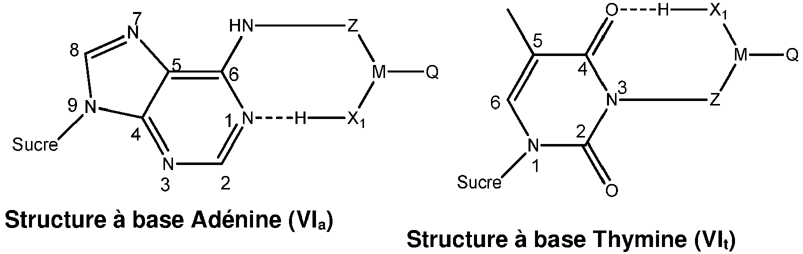 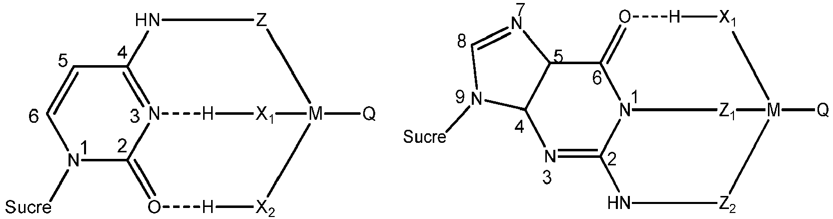 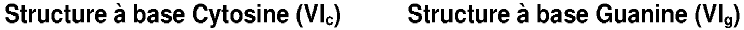 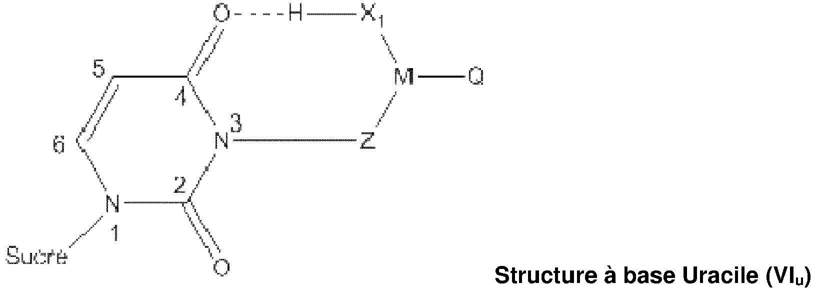 dans lesquelles :
« Sucre » représente la liaison entre ladite base azotée et la molécule de ribose ou de désoxyribose de la molécule de nucléotide, Z₁ et Z₂ sont des groupes Z clivables, identiques ou différents,
X₁ et X₂, identiques ou différents, représentent des atomes d'azote, d'oxygène ou de soufre portés par M et aptes à former avec lesdites bases azotées du nucléotide modifié des liaisons hydrogène intermoléculaires (similaires à celles observées lors des appariements classiques entre nucléotides complémentaires).

2. Nucléotide selon la revendication 1, utilisable comme substrat de polymérases normalement dépendantes de la présence d'un brin d'acide nucléique matrice complémentaire du brin en cours de synthèse, même en absence d'un brin complémentaire.

3. Nucléotide selon l'une quelconque des revendications précédentes, **caractérisé en ce que** le radical Q terminal fonctionnel du groupement R est apte à interagir avec des molécules autres qu'un acide nucléique selon l'un ou l'autre des couples d'interactions suivants : antigène/anticorps, hormone/récepteur, biotine/(strept)avidine, neurotransmetteur/récepteur, polymérase/promoteur, digoxine/antidigoxine, glucide/lectine, radical sulfuré/métal tel que l'or, glutathion/glutathionne S-transférase, ligand bidentate/oxyde métallique.

4. Nucléotide selon la revendication 1 pour lequel X₁ et X₂ sont -NH, T est -NH₂, Z est -CH₂, M est nitrophenyl-, et Q est -biotine.

5. Nucléotide selon l'une quelconque des revendications précédentes, **caractérisé en ce que** T et Z, ou Z₁, Z₂ sont clivables, lors de la synthèse d'acide nucléique, par irradiation dudit nucléotide au moyen de rayonnements électromagnétiques de longueur d'onde comprise entre 10⁻³ et 10⁻¹¹ mètres, notamment par des rayonnements ultra-violets.

6. Utilisation de nucléotide selon l'une quelconque des revendications précédentes dans un procédé de production de gènes, de séquence d'acides nucléiques synthétiques, d'ADN, d'ARN ou de polymères d'acides nucléiques, selon un procédé de synthèse enzymatique.

7. Utilisation de nucléotide selon la revendication 6, pour l'incorporation par voie enzymatique dudit nucléotide dans une chaîne polynucléotidique préalablement immobilisée sur un support solide.

8. Utilisation de nucléotide selon la revendication 7, **caractérisée en ce que** la chaîne polynucléotidique est fixée par son extrémité 5' et l'incorporation dudit nucléotide est réalisée par l'extrémité 3' de la chaîne de polynucléotide.

9. Kit pour la synthèse d'acide nucléique comprenant au moins un nucléotide modifié selon l'une quelconque des revendications 1 à 5.

10. Kit selon la revendication 9 comprenant différents nucléotides modifiés selon l'une quelconque des revendications 1 à 5, un enzyme d'élongation et un support solide apte à fixer au moins l'un des dits nucléotides.

## Patentansprüche

1. Verändertes Nukleotid zur enzymatischen Synthese von Nukleinsäuren, umfassend eine "natürliche" Stickstoffbase, ein Ribose- oder Desoxyribosekohlenhydrat und wenigstens eine Phosphatgruppe,
**dadurch gekennzeichnet, dass** es wenigstens eine Gruppe R umfasst, die als modifizierende Gruppe bezeichnet und von der "natürlichen" Stickstoffbase getragen ist, wobei R wenigstens eine funktionelle Endgruppe umfasst, wobei das Nukleotid in der folgenden Formel (III) vorliegt: 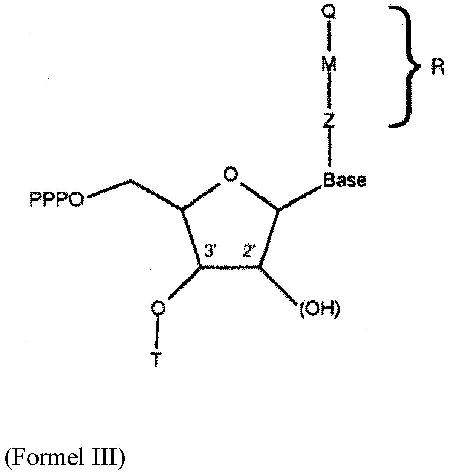 in der:
PPPO für eine Triphosphatgruppe steht,
(OH) die Möglichkeit eines Ribose- oder Desoxyribosemoleküls beschreibt,
T ein Wasserstoff oder ein spaltbarer Rest ist, der aus -NH₂, -N₃, -(C=O)H, -CₙH₂ₙ₊₁, wobei n zwischen 1 und 30 ist, vorzugsweise zwischen 1 und 12 ist, -Trimethylsilyl, -Phosphat, -SO₃, - (C=O)OCₙH₂ₙ₊₁, wobei n zwischen 1 und 30 ist, vorzugsweise zwischen 1 und 12 ist, - (C=O)SCₙH₂ₙ₊₁, wobei n zwischen 1 und 30 ist, vorzugsweise zwischen 1 und 12 ist, -Nitrobenzol, -Benzyl, -Halogenbenzyl, -Amid, -Carbonat, -Benzoyl, -Peroxyl, -Nitril, -Thiol, -Imid, -Carbamat, Cyanat, -Alkin, - Phenyl, -Halogenphenyl, -Picolyl ausgewählt ist,
M eine Gruppe ist, die mit Q und Z kovalent verbunden ist, wobei M aus Alkyl, Alkenyl, Alkin, Aryl, Alkylaryl, Heteroaryl, Acyl, Alkyloxy, Alkylamino, Alkoxyamino, Amido, Alkylimido, Alkenylimido, Arylimido, Fluoralkyl, Alkylphosphat, Alkylthio, Thioacyl, Alkylsulfonyl, Arylsulfonyl, Alkylsulfinyl, Alkylammonium, Alkylsulfonium, Alkylsilyl, Alkylcarbonyl, Alkylcarbanyl, Alkylcarbamoyl oder Alkylhydroxylamino ausgewählt ist,
Z eine spaltbare Gruppe ist, die aus -O-, -S-, =SH-, =S=, ≡S-, -SiH₂-, =SiH-, =Si=, ≡Si-, -Se-, =SeH-, ≡Se-, =Se=, -SeH₂-, -PH-, =P-, =PH=, ≡P=, ≡PH-, -PH₃-, -AsH-, =As-, =AsH=, ≡As=, ≡AsH-, -AsH₃-, Amin, Ester, Silyl, Alkyl, Benzyl, Nitrobenzyl, Amid, Carbonat, Benzoyl, Peroxyl, Nitril, Thiol, Imid, Carbamat, Cyanat, Hydroxylamin, Sulfoxid, Sulfonat, Thiosulfinat, Thioester, Acylhalogenid, Hypoiodyl, Alkin, Halogenphenyl, Halogenbenzyl, Picolyl, Diol oder Disulfid ausgewählt ist oder aus -CH₂ oder -NH- ausgewählt ist, wenn M ein -Nitrobenzyl-, ein - Nitrotolyl, ein -Nitroxylyl-, ein -Nitronaphthyl- oder ein -Nitrophenyl- ist,
Q eine terminale funktionelle Gruppe oder Effektorgruppe der Gruppe R ist, wobei Q aus Biotin, einem Protein, einem Polynukleotid definierter Sequenz, einem Kohlenhydrat, einem Antigen, einem Hormon, einem Neurotransmitter, einem Glycosid wie Digoxin, einem schwefelhaltigen Rest oder einem zweizähnigen Liganden wie Catechol ausgewählt ist,
"Base" für eine "natürliche" Stickstoffbase steht, die aus Adenin, Thymin, Cytosin, Guanin oder Uracil ausgewählt ist,
die modifizierende Gruppe R, die von der Stickstoffbase getragen ist, eine der folgenden Strukturen (VI) bildet: 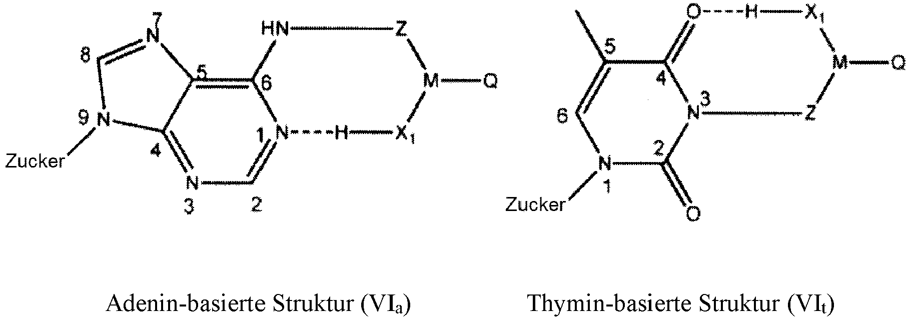 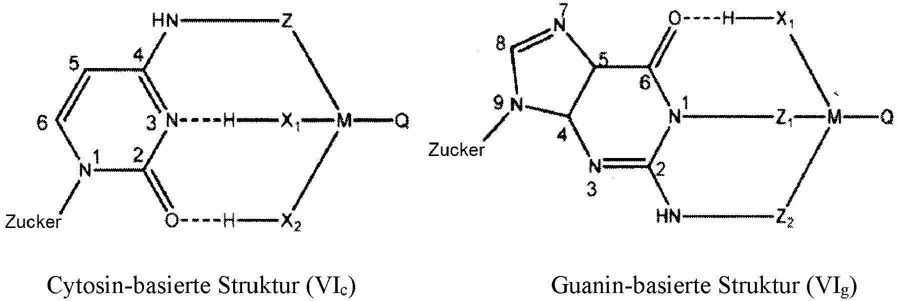 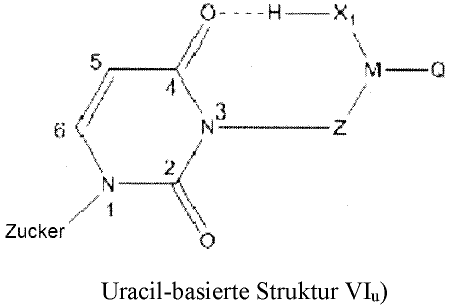 in denen:
"Zucker" für die Bindung zwischen der Stickstoffbase und dem Ribose- oder Desoxyribosemolekül des Nukleotidmoleküls steht, Z₁ und Z₂ spaltbare Z-Gruppen sind, die identisch oder verschieden sein können,
X₁ und X₂, die identisch oder verschieden sein können, für Stickstoff-, Sauerstoff- oder Schwefelatome stehen, die an M gebunden sind und in der Lage sind, mit den Stickstoffbasen des veränderten Nukleotids intermolekulare Wasserstoffbrücken-Bindungen zu bilden (ähnlich denjenigen, die bei herkömmlichen Paarungen zwischen komplementären Nukleotiden beobachtet werden).

2. Nukleotid gemäß Anspruch 1, das als Substrat für Polymerasen, die normalerweise von dem Vorhandensein eines Template-Nukleinsäurestrangs, der zum Strang komplementär ist, während der Synthese abhängig sind, selbst in Abwesenheit eines Komplementärstrangs verwendet werden kann.

3. Nukleotid gemäß einem der vorhergehenden Ansprüche, **dadurch gekennzeichnet, dass** der funktionelle terminale Rest Q der Gruppe R in der Lage ist, mit anderen Molekülen als einer Nukleinsäure gemäß einem der folgenden Interaktionspaare zu interagieren: Antigen/Antikörper, Hormon/Rezeptor, Biotin/(Strept)avidin, Neurotransmitter/Rezeptor, Polymerase/Promotor, Digoxin/Antidigoxin, Kohlenhydrat/Lectin, schwefelhaltiger Rest/Metall wie Gold, Glutathion/Glutathion-S-Transferase, zweizähniger Ligand/Metalloxid.

4. Nukleotid gemäß Anspruch 1, wobei X₁ und X₂ -NH sind, T -NH₂ ist, Z -CH₂ ist, M Nitrophenyl- ist und Q -Biotin ist.

5. Nukleotid gemäß einem der vorhergehenden Ansprüche, **dadurch gekennzeichnet, dass** T und Z oder Z₁, Z₂ während der Nukleinsäuresynthese durch Bestrahlung des Nukleotids mit elektromagnetischen Strahlen mit einer Wellenlänge zwischen 10⁻³ und 10⁻¹¹ Meter, insbesondere mit ultravioletten Strahlen gespaltet werden können.

6. Verwendung des Nukleotids gemäß einem der vorhergehenden Ansprüche in einem Verfahren zur Herstellung von Genen, von synthetischen Nukleinsäuresequenzen, von DNA, von RNA oder von Nukleinsäurepolymeren nach einem enzymatischen Syntheseverfahren.

7. Verwendung des Nukleotids gemäß Anspruch 6, zum enzymatischen Einbau des Nukleotids in eine Polynukleotidkette, die zuvor auf einem festen Träger immobilisiert wird.

8. Verwendung des Nukleotids gemäß Anspruch 7, **dadurch gekennzeichnet, dass** die Polynukleotidkette mit ihrem 5'-Ende fixiert wird und der Einbau des Nukleotids am 3'-Ende der Polynukleotidkette erfolgt.

9. Kit zur Synthese von Nukleinsäure, umfassend wenigstens ein Nukleotid, das gemäß einem der Ansprüche 1 bis 5 verändert ist.

10. Kit gemäß Anspruch 9, umfassend verschiedene Nukleotide, die gemäß einem der Ansprüche 1 bis 5 verändert sind, ein Enzym zur Elongation und einen festen Träger, der zur Fixierung wenigstens eines der Nukleotide geeignet ist.

## Claims

1. A modified nucleotide, intended for the enzymatic synthesis of nucleic acids, comprising a "natural" nitrogenous base, a ribose or deoxyribose carbohydrate, and at least one phosphate group,
**characterized in that** it comprises at least one R group, called modifier group, borne by said natural nitrogenous base, R comprising at least one functional end group, said nucleotide being in the form of (III)below: 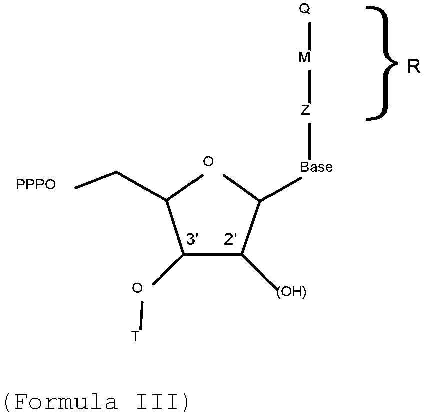 in which:
PPPO represents a triphosphate group,
(OH) describes the possibility of a ribose or deoxyribose molecule,
T is a hydrogen, or a cleavable radical chosen from -NH₂, -N₃, - (C=O)H, -CₙH₂ₙ₊₁ with n between 1 and 30, preferably between 1 and 12, -trimethylsilyl, -phosphate, -SO₃, -(C=O)OCₙH₂ₙ₊₁ with n between 1 and 30, preferably between 1 and 12, -(C=O)SCₙH₂ₙ₊₁ with n between 1 and 30, preferably between 1 and 12, -nitrobenzene, -benzyl, -halobenzyl, -amide, -carbonate, -benzoyl, -peroxyl, - nitrile, -thiol, -imide, -carbamate, -cyanate, -alkyne, - phenyl, -halophenyl, -picolyl,
M is a group covalently bonded to Q and to Z, M being chosen from alkyl, alkenyl, alkyne, aryl, alkylaryl, heteroaryl, acyl, alkyloxy, alkylamino, alkoxyamino, amido, alkylimido, alkenylimido, arylimido, fluoroalkyl, alkylphosphate, alkylthio, thioacyl, alkylsulfonyl, arylsulfonyl, alkylsulfinyl, alkylammonium, alkylsulfonium, alkylsilyl, alkylcarbonyl, alkylcarbonyl, alkylcarbanyl, alkylcarbamoyl or alkylhydroxylamino,
Z is a cleavable group, chosen from -O-, -S-, =SH-, =S=, ≡S-, - SiH₂-, =SiH-, =Si=, ≡Si-, -Se-, =SeH-, ≡Se-, =Se=, -SeH₂-, -PH-, =P-, =PH=, ≡P=, ≡PH-, -PH₃-, -AsH-, =As-, =AsH=, ≡As=, ≡AsH-, -ASH₃-, amine, ester, silyl, alkyl, benzyl, nitrobenzyl, amide, carbonate, benzoyl, peroxyl, nitrile, thiol, imide, carbamate, cyanate, hydroxylamine, sulfoxide, sulfonate, thiosulfinate, thioester, acyl halide, hypoiodyl, alkyne, halophenyl, halobenzyl, picoyl, diol or disulfide, or chosen from -CH₂ or - NH- when M is a -nitrobenzyl-, a -nitrotolyl-, a -nitroxylyl-, a -nitronaphthyl- or a -nitrophenyl-,
Q is a functional, or effector, end group of the R group, Q being chosen from biotin, a protein, a polynucleotide of defined sequence, a carbohydrate, an antigen, a hormone, a neurotransmitter, a glycoside such as digoxin, a sulfur-containing radical, or a bidentate ligand such as catechol, "base" represents a "natural" nitrogenous base chosen from adenine, thymine, cytosine, guanine or uracil,
the modifier group R borne by the nitrogenous base forms one of the structures (VI) below: 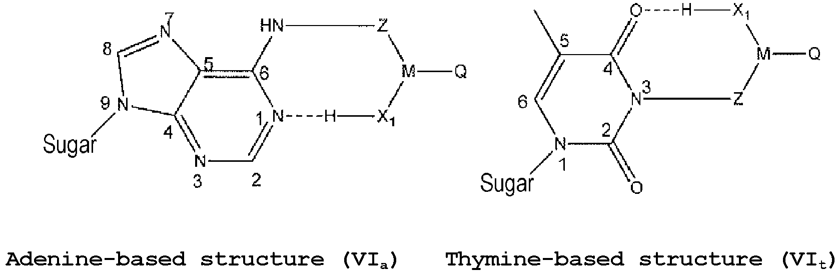 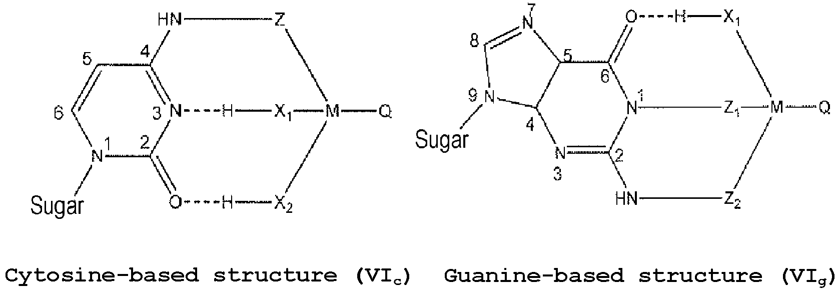 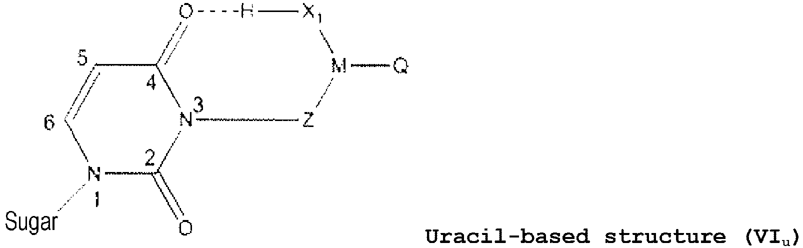 in which:
"Sugar" represents the bond between the nitrogenous base and the ribose or deoxyribose molecule of the nucleotide molecule, Z₁ and Z₂ are identical or different cleavable group Z. X₁ and X₂, which may be identical or different, represent nitrogen, oxygen or sulfur atoms borne by M and capable of forming, with said nitrogenous bases of the modified nucleotide, intermolecular hydrogen bonds (similar to those observed during conventional pairings between complementary nucleotides).

2. The nucleotide according to claim 1, usable as a substrate for polymerases that are normally dependent on the presence of a template nucleic acid strand complementary to the strand undergoing synthesis, even in the absence of a complementary strand.

3. The nucleotide according to any one of the preceding claims, **characterized in that** the functional end radical Q of the R group is capable of interacting with molecules other than a nucleic acid according to one or another of the following interaction pairs: antigen/antibody, hormone/receptor, biotin/(strept)avidin, neurotransmitter/receptor, polymerase/promoter, digoxin/antidigoxin, carbohydrate/lectin, sulfur-containing radical/metal such as gold, glutathione/glutathione S-transferase, bidentate ligand/metal oxide.

4. The nucleotide according to claim 1, for which X₁ and X₂ are -NH, T is -NH₂, Z is -CH₂, M is nitrophenyl-, and Q is -biotin.

5. The nucleotide according to any one of the preceding claims, **characterized in that** T and Z, or Z₁, Z₂ are cleavable, during the nucleic acid synthesis, by irradiation of said nucleotide by means of electromagnetic radiation having a wavelength of between 10⁻³ and 10⁻¹¹ meter, in particular by ultraviolet radiation.

6. The use of a nucleotide according to any one of the preceding claims in a process for the production of genes, of synthetic nucleic acid sequences, of DNA, of RNA or of nucleic acid polymers, according to an enzymatic synthesis process.

7. The use of a nucleotide according to claim 6, for incorporating by enzymatic process said nucleotide into a polynucleotide chain previously immobilized on a solid support.

8. The use of a nucleotide according to claim 7, **characterized in that** the polynucleotide chain is attached via its 5' end and the incorporation of said nucleotide is carried out via the 3' end of the polynucleotide chain.

9. A kit for nucleic acid synthesis, comprising at least one modified nucleotide as claimed in any one of claim 1 to 5

10. The kit according to claim 9, comprising various modified nucleotides according to any one of claim 1 to 5, an elongation enzyme and a solid support capable of attaching at least one of said nucleotides.


### SEQUENCE LISTING

<110> DNA SCRIPT DNA SCRIPT
<120> Nucléotides modifiés
<130> PN000534
<150> FR1458194
   <151> 2014-09-02
<160> 2
<170> PatentIn version 3.5
<210> 1
   <211> 20
   <212> DNA
   <213> acide nucléique
<400> 1
   agccaagcgg tcgcgatgat 20
<210> 2
   <211> 12
   <212> DNA
   <213> acide nucléique
<400> 2
   gtccgcttgg ct 12